# FASCICULE DE BREVET EUROPEEN

(11) **EP 0 283 327 B1**
(45) Date de publication et mention de la délivrance du brevet: **25.06.1997**
(21) Numéro de dépôt: 88400084.5
(22) Date de dépôt: 15.01.1988
(51) Int. Cl.: C07K 14/155, G01N 33/569, A61K 39/21, A61K 38/02

(54) **Peptides ayant des propriétés immunologiques de HIV-2**
Peptide mit den immunologischen Eigenschaften von HIV-2
Peptides with the immunological properties of HIV-2

(30) Priorité: 16.01.1987 US 3764; 11.02.1987 FR 8701739; 15.04.1987 FR 8705398
(43) Date de publication de la demande: 21.09.1988
(60) Demande divisionnaire: 96108720.2
(73) Titulaire: INSTITUT PASTEUR, F-75015 Paris (FR); CENTRE NATIONAL DE LA RECHERCHE SCIENTIFIQUE, 75007 Paris (FR)
(72) Inventeur: Alizon, Marc, F-75005 Paris (FR); Montagnier, Luc, F-92350 Le Plessis Robinson (FR); Guetard, Denise, F-75015 Paris (FR); Clavel, François, Rockville MD 20852 (US); Sonigo, Pierre, F-75015 Paris (FR); Guyader, Mireille, F-75017 Paris (FR); Tiollais, Pierre, F-75013 Paris (FR); Chakrabarti, Lisa, F-75005 Paris (FR); Desrosiers, Ronald, Hudson, MA 01749 (US)
(74) Mandataire: Gutmann, Ernest

(56) Documents cités:
- EP-A- 0 187 041
- EP-A- 0 199 301
- EP-A- 0 239 425
- EP-A- 0 269 520
- WO-A-86/02383
- WO-A-87/02038
- FR-A- 2 593 189
- US-A- 4 629 783
- SCIENCE, vol. 232, 1985, pages 238-243
- SCIENCE, vol. 233, 18 juillet 1986, pages 343-346
- NATURE, vol. 324, 18/25 décembre 1986, pages 691-695, MacMillan Eds., London, GB
- NATURE, vol. 321, 22 mai 1986, pages 435-437, MacMillan Eds., London, GB
- JOURNAL OF CELLULAR BIOCHEMISTRY, suppl. 11D, 1987, Abstracts 16th Annual Meetings, 1-6 avril 1987, page 44, abrégé P112, A.R. Liss Inc., New York, US
- NATURE, vol. 326, no. 6113, 9/15 avril 1987, pages 610-613, MacMillan Eds., London, GB
- NATURE, vol. 326, 16 avril 1987, pages 662-669, MacMillan Eds., London, GB
- FEBS LETTERS, vol. 218, no. 2, juin 1987, pages 231-237, Elsevier Sc. Publ. B.V.

## Description

La présente invention est relative à des peptides ayant des propriétés immunologiques, le cas échéant immunogènes, en commun avec des antigènes susceptibles d'être obtenus sous une forme purifiée, à partir de virus capables de provoquer des lymphadénopathies susceptibles d'être relayées ensuite par le symdrôme d'immunodéficience acquise (SIDA) chez l'homme.

L'invention concerne en particulier des peptides antigéniques susceptibles d'être reconnus par des anticorps induits chez l'homme par des virus désignés par l'abréviation HIV, selon la nomenclature définie dans NATURE. Elle concerne également des peptides ayant des propriétés immunogènes ou susceptibles d'être rendus immunogènes in vivo, cette immunogénicité étant susceptible de se manifester par l'induction in vivo d'anticorps reconnaissant des antigènes caractéristiques des virus HIV-2 et même, au moins en ce qui concerne certains de ces peptides, des antigènes issus de HIV-1.

L'invention concerne en outre des applications de ces peptides à la fabrication de compositions pour le diagnostic in vitro chez l'homme de potentialité de certaines formes du SIDA et, en ce qui concerne certains d'entre eux, à la production de compositions immunogènes et de compositions vaccinantes contre les rétrovirus HIV.

De même l'invention concerne les applications aux mêmes fins des anticorps susceptibles d'être induits in vivo par les peptides immunogènes ou rendus immunogènes et, pour certains de ces anticorps, leurs applications à la production de principes actifs de médicaments contre ces SIDAS humains.

L'invention concerne également la mise en oeuvre de certains de ces peptides dans des procédés pour le diagnostic in vitro chez l'homme de certaines formes du SIDA, ainsi que leur application à la constitution de trousses ou "kits" de diagnostic.

Un premier rétrovirus dénommé LAV-1 ou HIV-1 a été isolé et décrit dans la demande de brevet GB.83/24.800 et une demande EP.84/401.834 du 14/09/84. Ce virus a également été décrit par F.Barre Sinoussi et al. dans Science, 220 n° 45-99, 20 pages 868-871.

Des variants de ce virus HIV-1 désignés par LAV ELI et LAV MAL, ont également été isolés, caractérisés et décrits dans la demande de brevet EP.84/401.834.

Les virus HIV-1 et leurs variants possèdent les propriétés suivantes :
- ils ont pour cibles préférencielles les cellules Leu3 (ou lymphocytes T4) humaines et leurs cellules dérivées "immortalisées".
- ils ont une activité transcriptase inverse nécessitant la présence d'ions Mg²⁺ et présentent une forte activité pour le poly(adenylate-oligo-deoxythymidylase) poly(A)-oligo(dT)12-18)
- ils ont une densité de 1,16 à 1,17 sur gradient de sucrose,
- ils ont un diamètre moyen de 139 nanomètres et un noyau de diamètre moyen de 41 nanomètres,
- les lysats de ces virus contiennent une protéine p25 (protéine du noyau) qui ne croise pas immunologiquement avec la protéine p24 de HTLV-1,
- ils contiennent une protéine p42 appartenant à leur enveloppe,
- ils contiennent également une glycoprotéine d'enveloppe gp110 d'un poids moléculaire de 110.000.

L'isolement et la caractérisation de rétrovirus appartenant à une classe distincte et n'ayant qu'une parenté immunologique réduite avec les précédents, ont été décrits dans la demande de brevet européen n° 87/400.151.4. Ces rétrovirus qui ont été regroupés sous la désignation HIV-2, ont été isolés chez plusieurs malades africains présentant des symptômes d'une lymphadénopathie ou d'un SIDA.

Les rétrovirus du type HIV-2 comme les rétrovirus du type HIV-1, se caractérisent par un tropisme pour les lymphocytes T4 humains et par un effet cytopathogène à l'égard de ces lymphocytes, lorsqu'ils s'y multiplient, pour alors causer soit des poly-adénopathies généralisées et persistantes, soit un SIDA.

Plus généralement les rétrovirus purifiés par HIV-2 possèdent en général les propriétés suivantes :
- la cible préférentielle des rétrovirus HIV-2 est constituée par les cellules Leu3 (ou lymphocytes T4) humaines et pour des cellules "immortalisées" dérivées de ces lymphocytes T4 ;
- ils sont cytotoxiques pour les lymphocytes T4 humains
- ils ont une activité de transcriptase inverse nécessitant la présence d'ions Mg²⁺ et présentant une forte activité pour le poly(adénylate-oligodéoxythylmidylase) (poly(A)-oligo(dT) 12-18) ;
- ils ont une densité de 1,16 dans un gradient de sucrose ;
- ils ont un diamètre moyen de 140 nanomètres et un noyau ayant un diamètre moyen de 41 nanomètres ;
- ils peuvent être cultivés dans des lignées permanentes du type HUT ou exprimant la protéine T4 ;
- ils ne sont pas infectieux pour les lymphocytes T8 ;
- les lysats de ces virus contiennent une protéine p26 qui ne croise pas immunologiquement avec la protéine p24 du virus HTLV-I ou du virus HTLV-II ;
- ces lysats contiennent en outre une protéine p16 qui n'est pas reconnue immunologiquement par la protéine p19 de HTLV-I ou de HTLV-II dans des essais de radioimmunoprécipitation ;
- ils contiennent en outre une glycoprotéine d'enveloppe ayant un poids moléculaire de l'ordre de 130.000-140.000 qui ne croise pas immunologiquement avec la gp110 des HIV-1, mais qui en revanche croise immunologiquement avec la glycoprotéine d'enveloppe gp140 de STLV-III (virus isolé chez le singe) ;
- ces lysats contiennent encore des antigènes marquables par la ³⁵S-cystéine, dont les poids moléculaires s'étagent entre 32.000 et 42.000-45.000 : ils comprennent notamment un antigène ayant un poids moléculaire de l'ordre de 36.000 et un antigène ayant un poids moléculaire de l'ordre de 42.000, l'un de ces antigènes (p36 et p42) constituant vraisemblablement une glycoprotéine transmembranaire du virus HIV-2 ;
- l'ARN génomique des HIV-2 n'hybride pas avec l'ARN génomique de HIV-1 dans des conditions stringentes ;
- dans des conditions non stringentes, l'ARN génomique de HIV-2 n'hybride, ni avec le gène env et le LTR qui le jouxte, de HIV-1, ni avec des séquences de la région pol du génome de HIV-1 ;
- dans des conditions non stringentes, il hybride faiblement avec des séquences de nucléotides de la région de HIV-1.

Un autre rétrovirus dénommé SIV-1, cette dénomination remplaçant la dénomination antérieurement connue STLV III, a été isolé chez le singe macaque rhésus. (M.D.Daniel et al. Science 228, 1201 (1985) N.L.Letwin et al, Science 230, 71 (1985) sous l'appellation "STLV-IIImac").

Un autre rétrovirus, désigné "STLV-III_{AGM}", (ou SIV_{AGM}) a été isolé chez des singes verts sauvages. Mais, contrairement au virus présent chez le singe macaque rhésus, la présence de "STLV-III_{AGM}" ne semble pas induire une maladie du type SIDA chez le singe vert d'Afrique.

Une souche du rétrovirus SIV-1mac a été déposée à la CNCM le 7 Février 1986 sous le n° I-521. Des études ont montré que le rétrovirus SIV-1 comporte certaines protéines possédant une certaine parenté immunologique avec des protéines ou glycoprotéines structurales susceptibles d'être obtenues dans des conditions analogues, à partir de HIV-2. Ce rétrovirus SIV-1, dont on a constaté le caractère infectieux chez les singes, avait été désigné par STLVIII par les chercheurs qui l'ont isolé (références bibliographiques précitées).

Pour la commodité du langage, ces virus ne seront plus désignés dans ce qui suit que par l'expression SIV (l'expression SIV est l'abréviation anglaise de "Simian Immunodeficiency Virus" (virus d'immunodéficience du singe)) éventuellement suivi d'une abréviation désignant l'espèce de singe dont ils sont issus, par exemple, MAC (ou mac) pour le macaque ou AGM pour le singe vert d'Afrique (abréviation de "African Green Monkey").

En mettant en oeuvre les mêmes techniques que celles rappelées plus haut, il a été constaté que l'on pouvait également obtenir à partir de SIV-1mac :
- une protéine principale du noyau p27, ayant un poids moléculaire de l'ordre de 27 kilodaltons,
- une glycoprotéine majeure d'enveloppe, gp140,
- une protéine vraisemblablement transmembranaire p32, qui n'est guère observée en RIPA lorsque le virus a au préalable été marqué par la ³⁵S-cystéine, mais qui peut être observée dans les essais d'immunoempreintes (Western blots), sous forme de bandes larges.

Des études plus précises ont été réalisées en ce qui concerne les précédents virus HIV-2 et SIV. La poursuite de l'étude des rétrovirus HIV-2 a également conduit à l'obtention de séquences d'ADN complémentaires (ADNc) des ARNs de leurs génomes. La séquence nucléotidique complète de l'ADNc d'un rétrovirus représentatif de la classe HIV-2 (HIV-2 ROD) a été déposée le 21/02/1986 à la CNCM sous le n° I-522, sous le nom de référence LAV-II ROD).

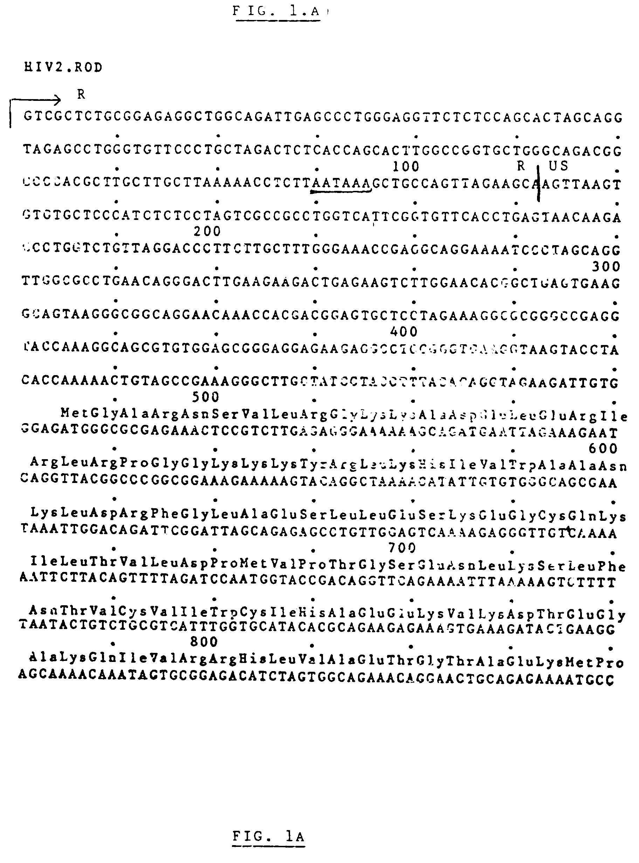

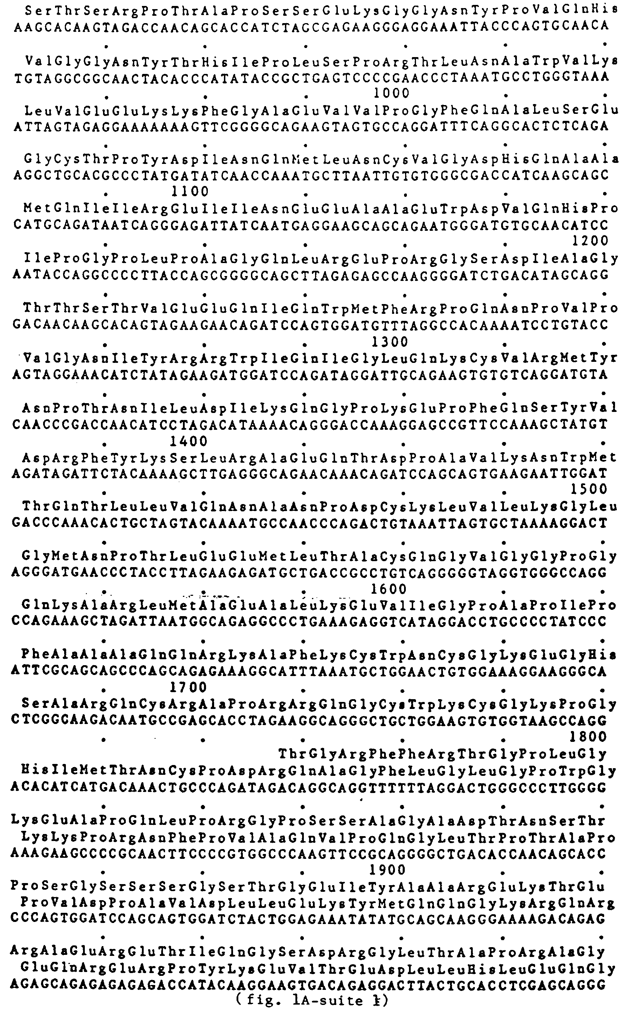

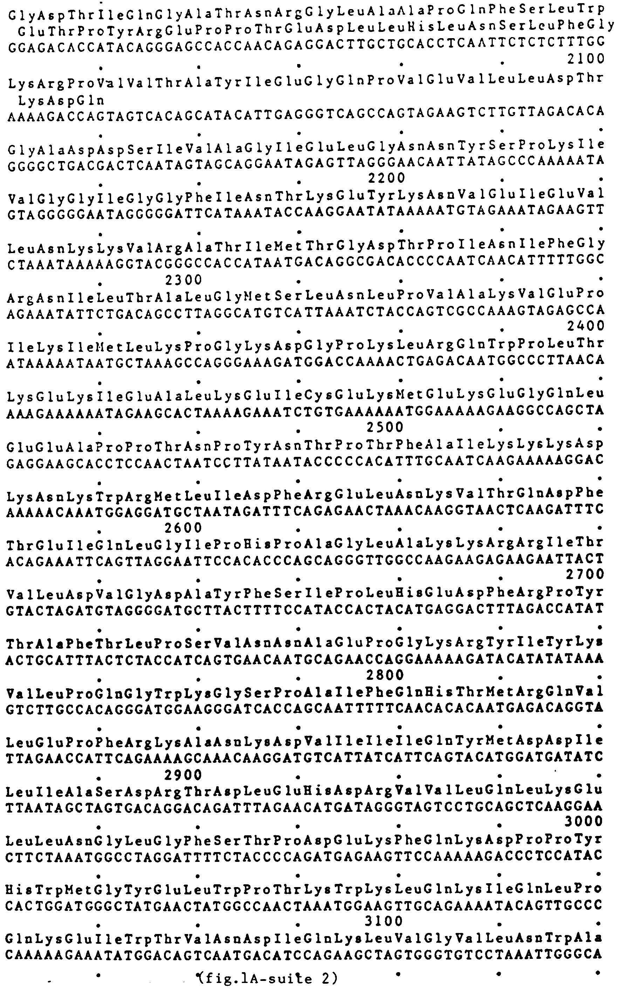

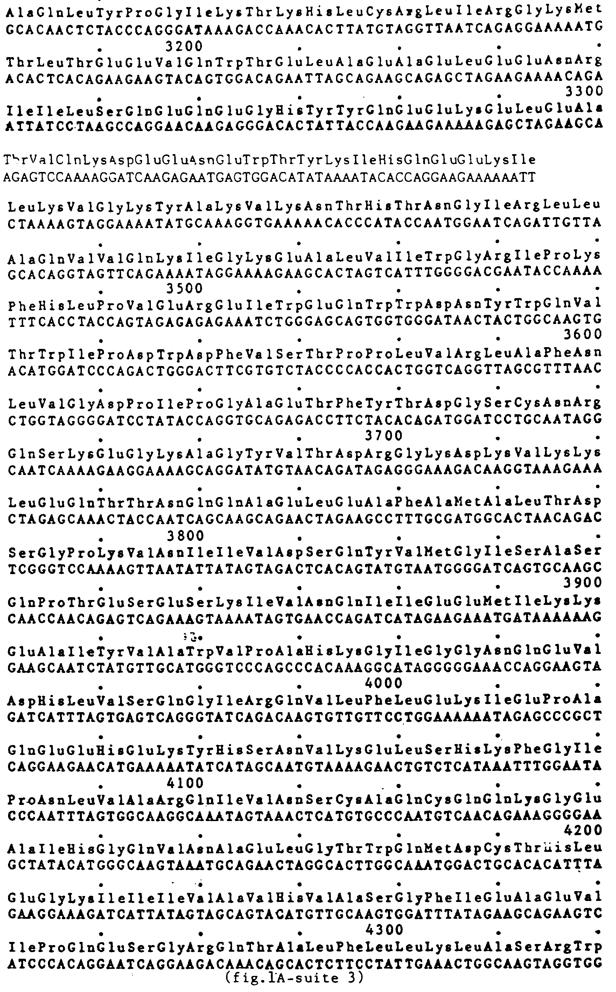

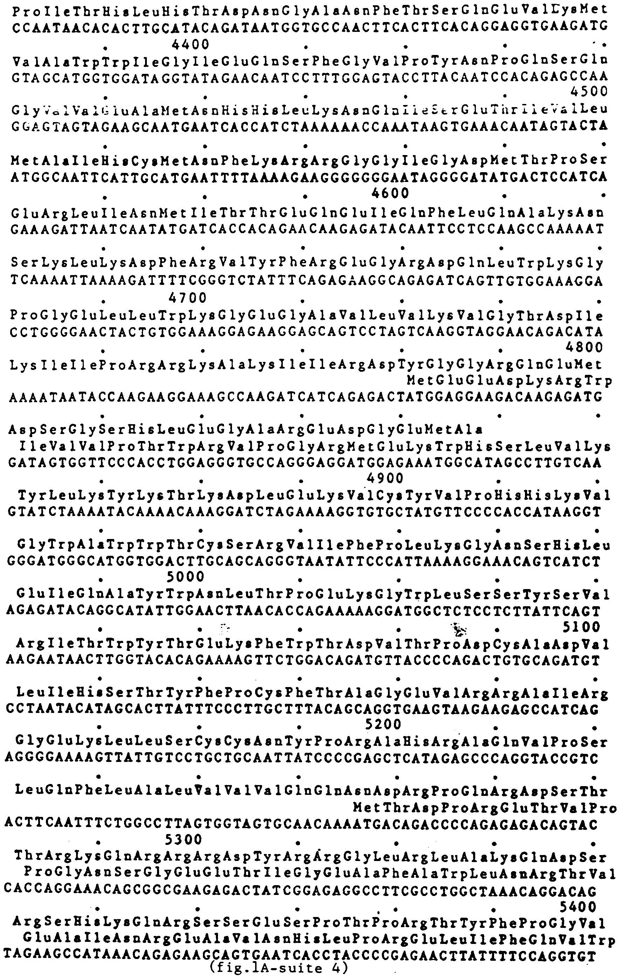

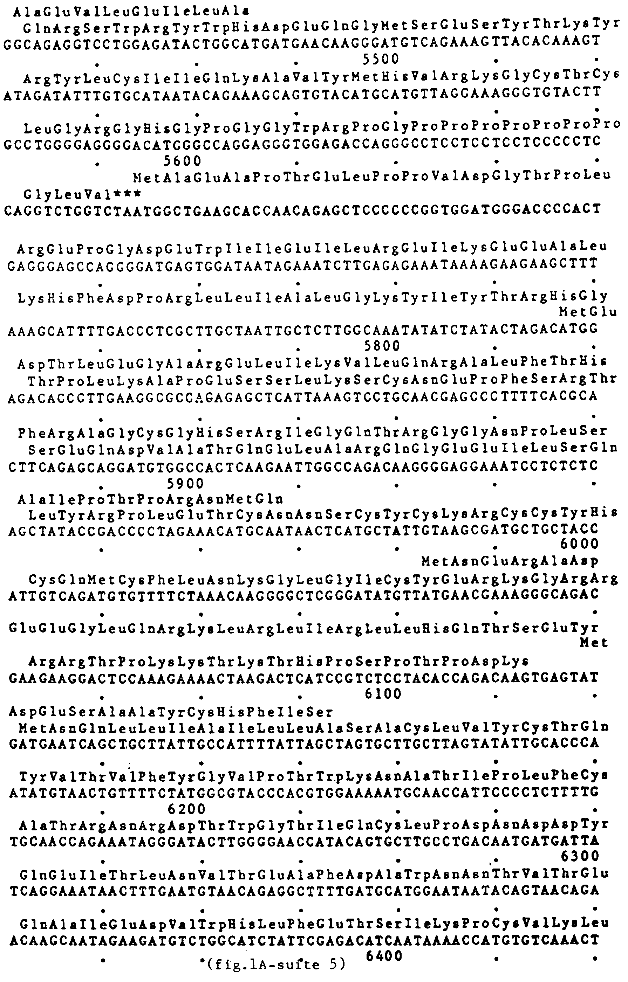

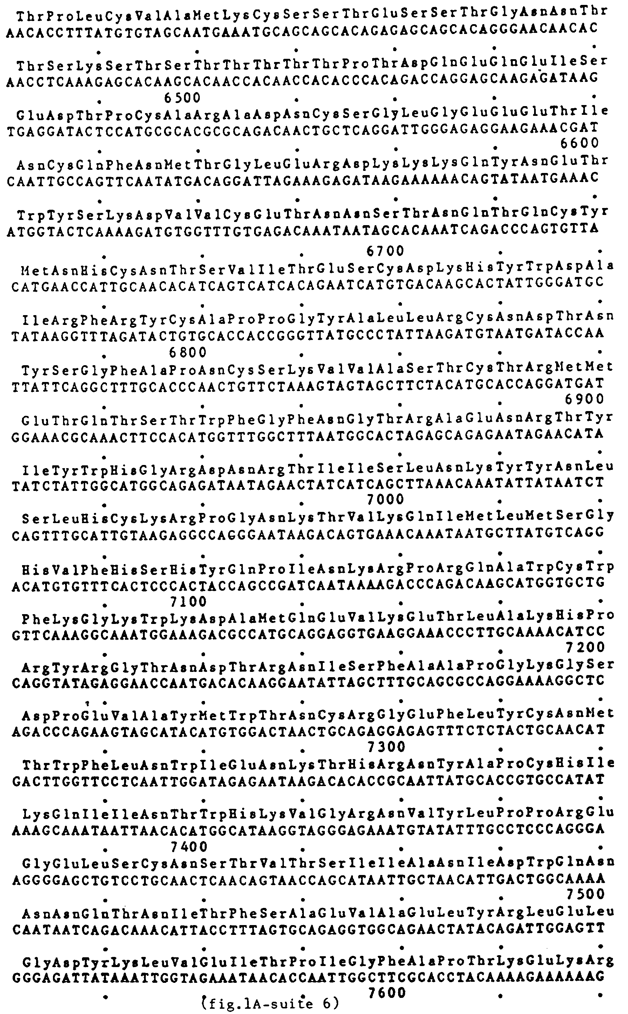

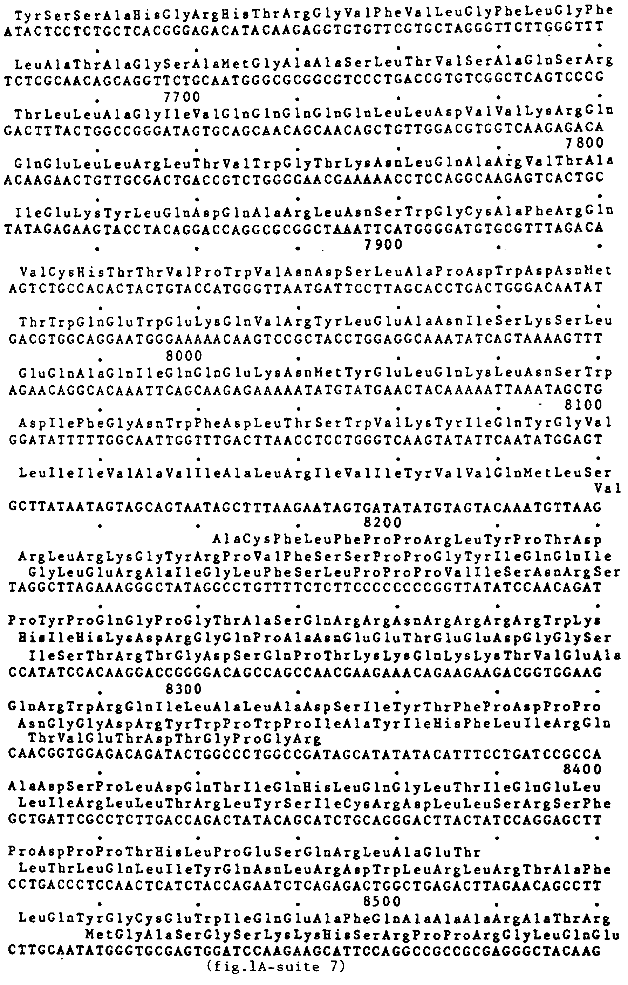

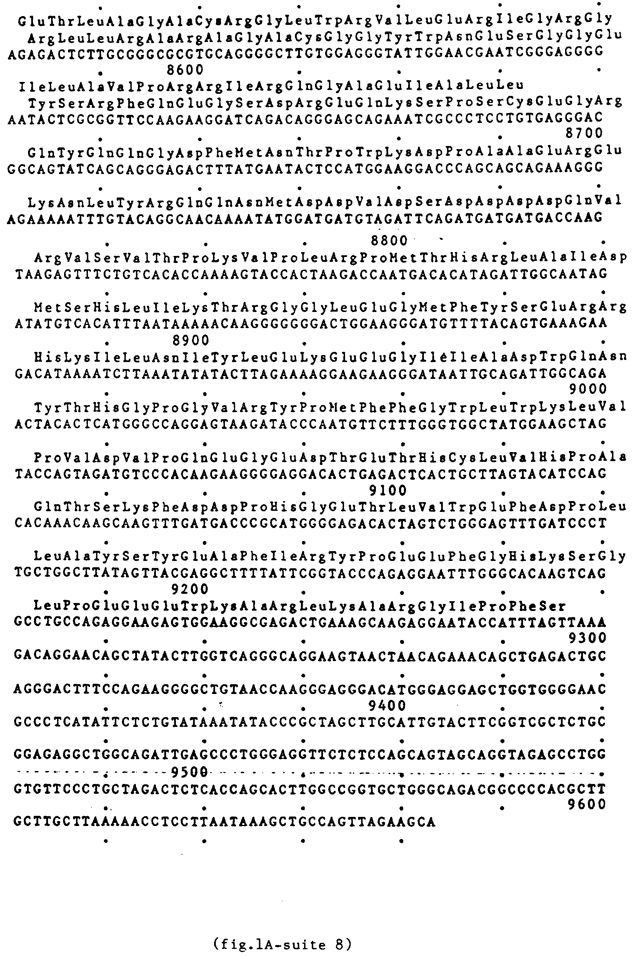

Cette séquence nucléotidique et les phases de lecture ouverte qu'elle contient sont indiqués à la figure 1 A.

En outre, la poursuite de l'étude d'autres rétrovirus a également permis d'aboutir à l'obtention de leurs séquences nucléotidiques complètes. Il en est en particulier ainsi de l'ADNc dérivé de l'ARN génomique de SIV.

Le clonage et le séquençage du virus SIV-1mac qui ont permis l'obtention de sa séquence nucléotidique ont été réalisés dans les conditions suivantes :

L'ADN de cellules HUT 78 infectées par le virus SIV (isolat STLV-III mac 142-83 décrit par Daniel et al.(1985) Science, 228, p. 1201-1204, digéré partiellement par l'enzyme de restriction Sau3A a été cloné au site BamHI du bactériophage vecteur Lambda ELBL3 pour constituer une banque génomique. Les 2 millions de phages recombinants de la banque génomique ainsi constituée ont été criblés in situ en conditions de sécurité P3, à l'aide de séquences du virus HIV2 provenant des clones lambda-ROD4, lambda-ROD35 et E2 (Clavel et al. (1986-Nature, 324, p.691.) et nick-translatées.

L'hybridation a été réalisée en 5xSSC à 50°C et les lavages en 2xSSC à 50°C. Un seul clone contenant l'ensemble des séquences virales a été obtenu. Ce clone est désigné par lambda-SIV-1. L'insérat du phage lambda-SIV-1 mesure 16,5 kb au total et comprend un provirus intégré auquel manquent seulement les 250 premières bases du LTR gauche, alors que le LTR droit est complet.

Le provirus intégré a été séquencé par la méthode des didéoxynucléotides après sous-clonage de fragments aléatoires dans le phage M13mp8. 300 sous-clones ont été analysés.

Des fragments d'ADNc provenant du clone Lambda SIV-1 insérés dans des plasmides pSIV-1.1 et pSIV-1.2 ont été déposés à la CNCM le 15 Avril 1987, sous les numéros I-658 (pSIV-1.1) et I-659 (pSIV-1.2).

Les résultats ont été mentionnés dans les figures décrites ci-après.

La figure 1B représente la séquence nucléotidique du génome viral de SIV et les séquences qui en sont déduites pour les protéines virales correspondant aux produits des gènes gag, pol, env, Q, X, R, tat, art, F.

Les figures 3 à 11 et la figure 1C représentent les comparaisons des produits théoriques des gènes viraux et des LTR entre HIV2 et SIVmac. (λSIV-1).

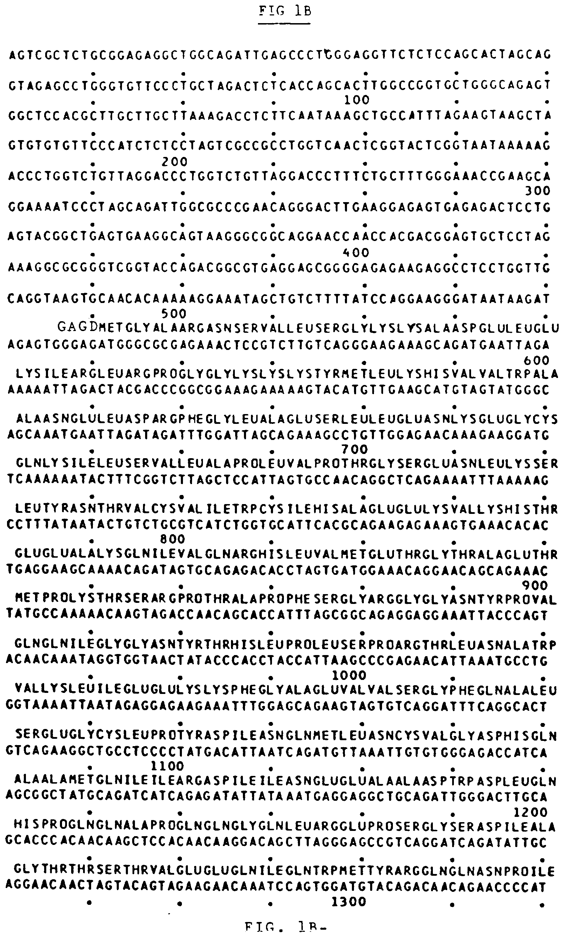

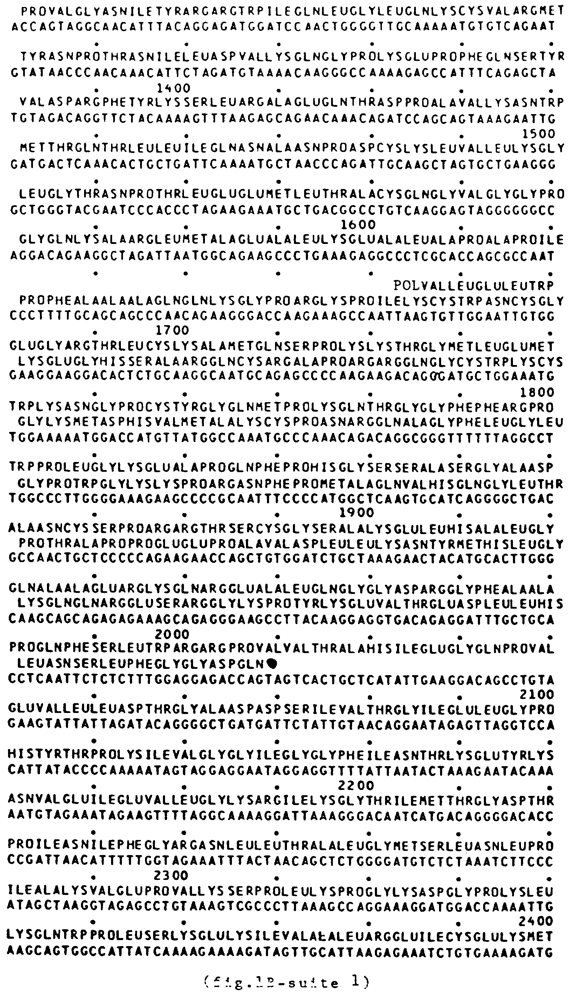

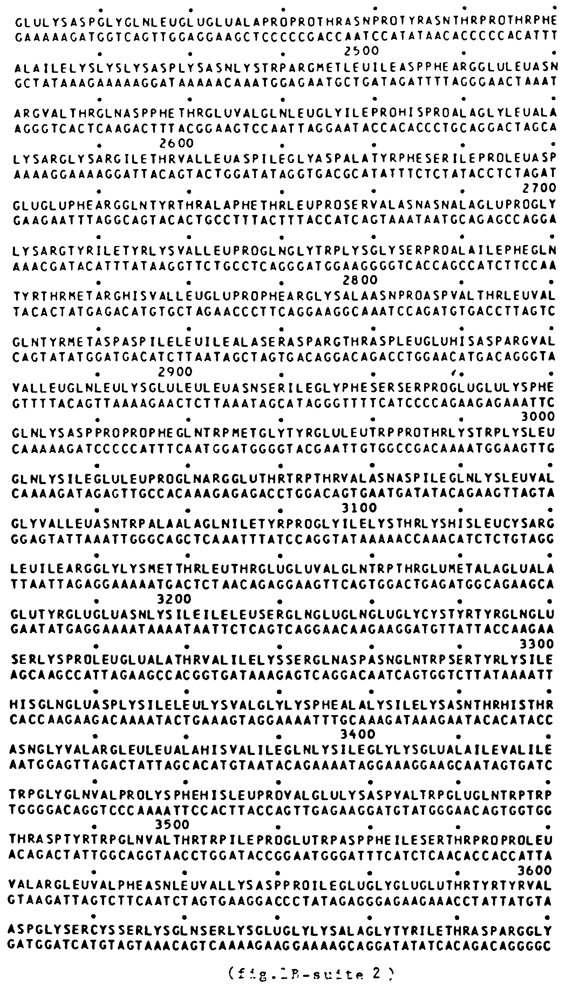

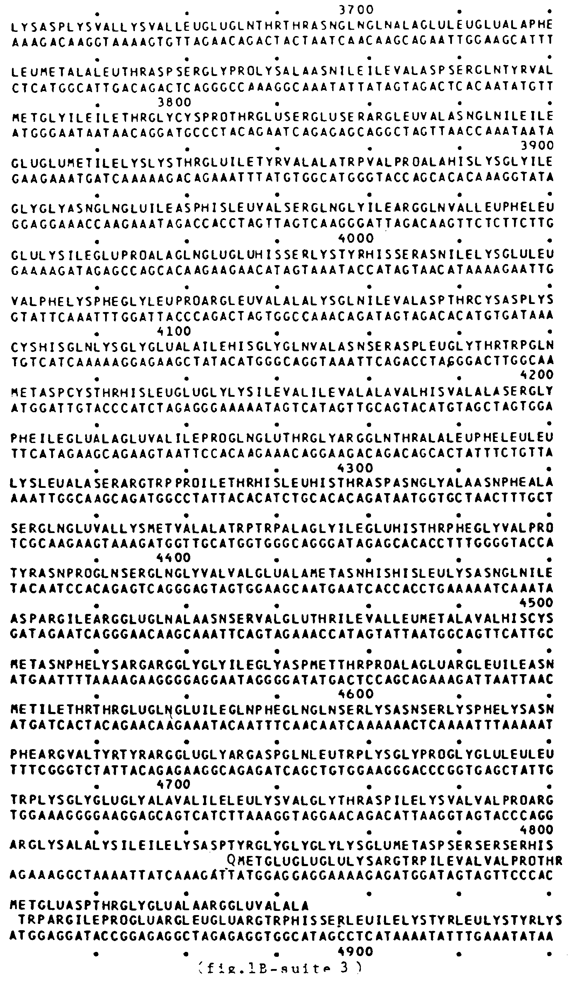

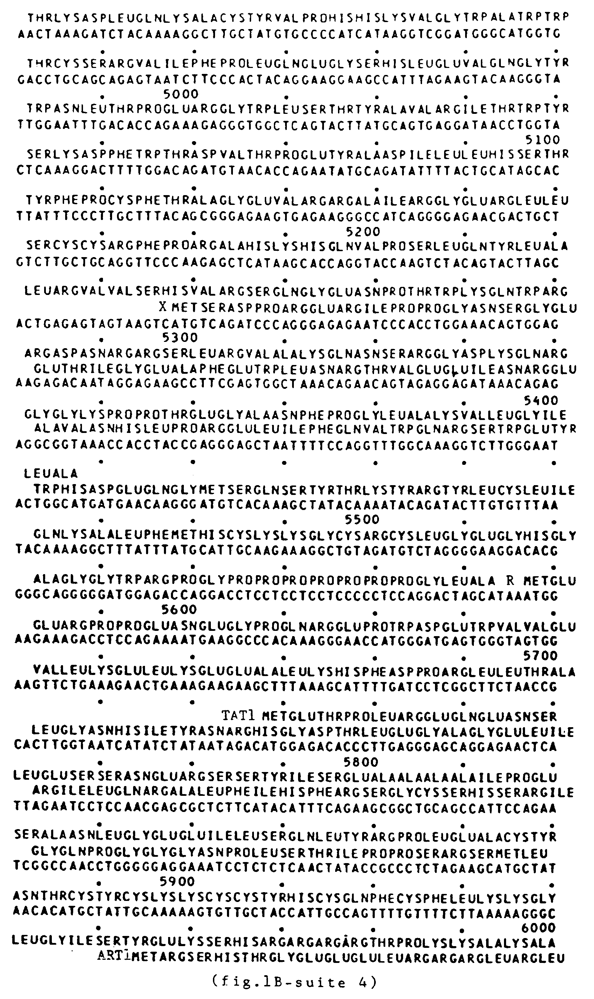

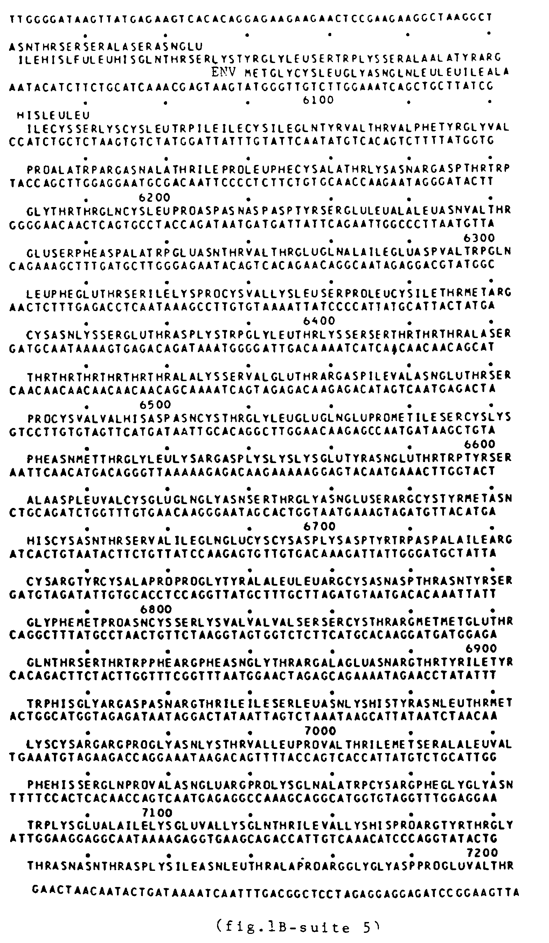

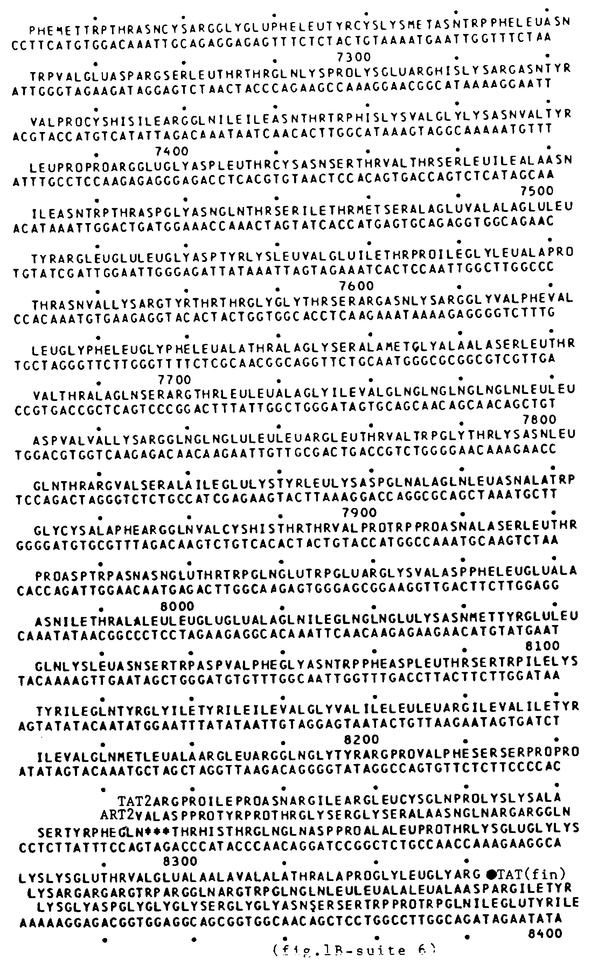

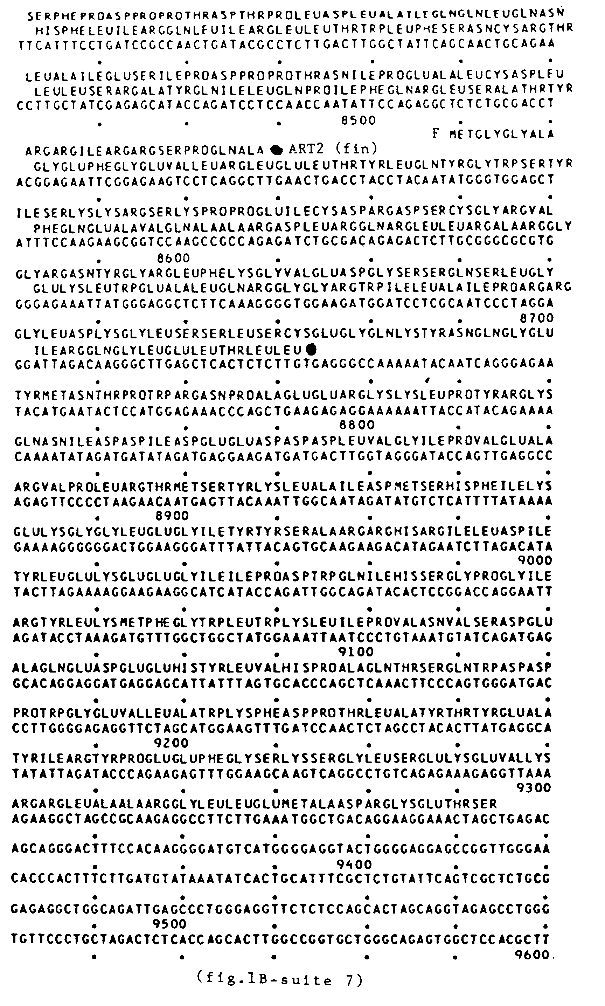

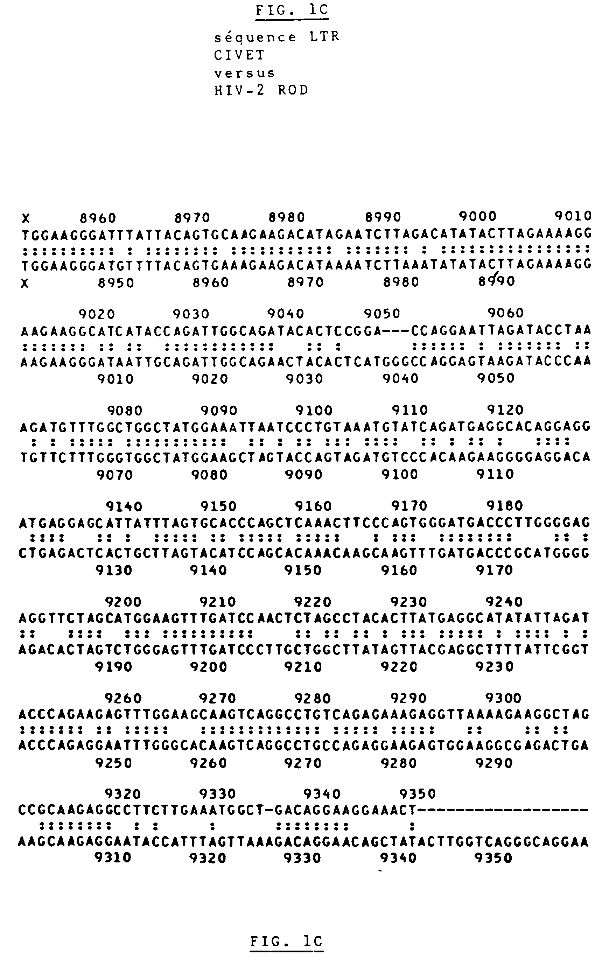

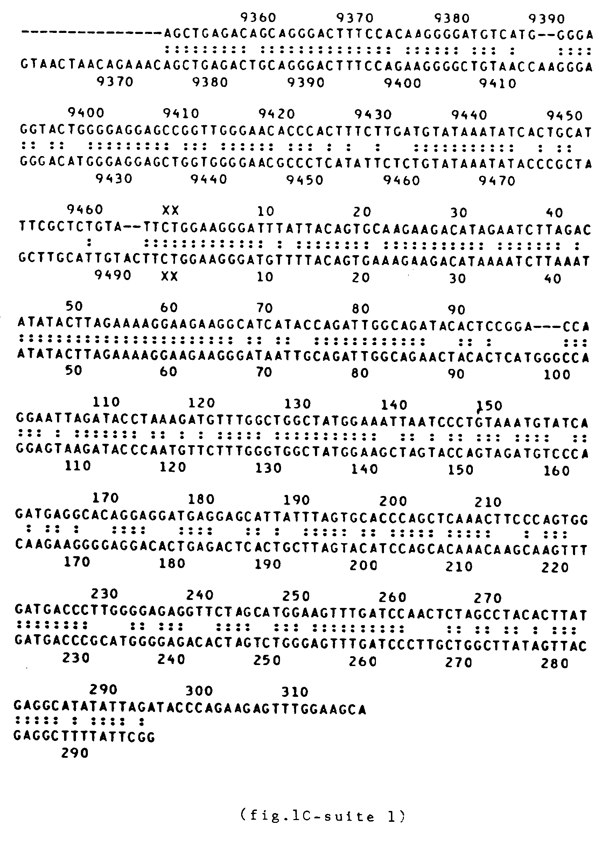

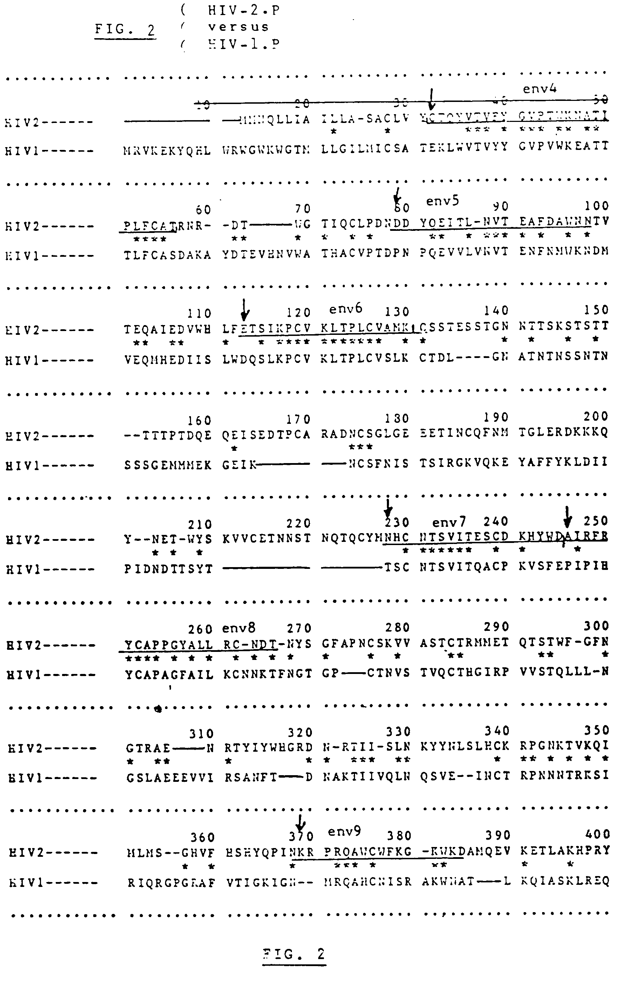

Les fragments d'ADNc déduits de l'ADNc issu du génome entier de SIV-1, qui contiennent une ou plusieurs séquences issues de la séquence complète d'ADNc et qui codent pour des peptides intéressants de l'invention sont également dècrits dans la présente demande. Ces sequences sont indiquées à la figure 1B et, à la figure 1C pour ce qui a trait à la séquence LTR du virus,

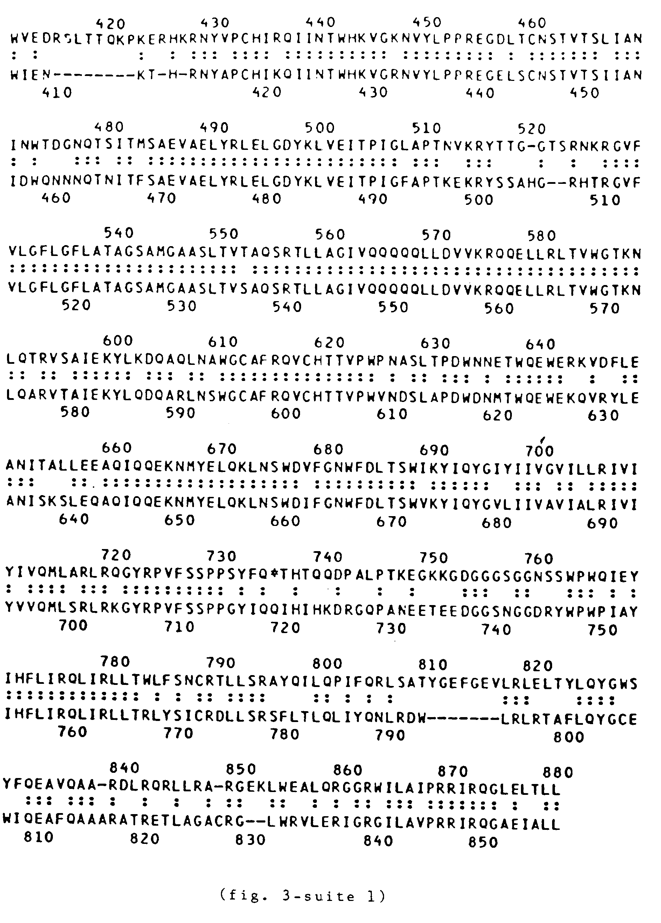

Les séquences nucléiques de l'ADNc de SIV ont été placées en correspondance avec les séquences nucléiques du virus HIV-2 ROD pour ce qui concerne la séquence LTR (figure 1C). Cette présentation que l'on retrouve pour le génome entier en rapprochant la figure 1B des figures 3 à 11 permet de repérer ou de déduire les acides nucléiques ayant des éléments de structure essentiels communs aux deux virus.

La présente demande dècrit aussi l'utilisation des cADNs issus de SIV ou de leurs fragments (ou de recombinants les contenant) en tant que sondes, pour le diagnostic de la présence ou non de virus HIV-2 dans des échantillons de sérums ou d'autres liquides ou tissus biologiques obtenus à partir de patients suspectés d'être porteurs du virus HIV-2. Ces sondes sont de préférence marquées également (marqueurs radio-actifs, enzymatiques, fluorescents, etc.). Des sondes particulièrement intéressantes pour la mise en oeuvre du procédé de diagnostic du virus HIV-2 ou d'un variant de HIV-2 peuvent être caractérisées en ce qu'elles comprennent la totalité ou une fraction de l'ADNc complémentaire du génome du virus SIV ou encore notamment les fragments recombinants contenus dans divers clones.

Les sondes utilisables dans ce procédé de diagnostic du virus HIV-2 et dans les kits de diagnostic ne sont en aucune façon réduites aux sondes décrites précédemment. Elles comprennent au contraire toutes les séquences nucléotidiques issues du génome du virus SIV, d'un variant de SIV ou d'un virus proche par sa structure, dès lors qu'elles permettent la détection dans des fluides biologiques de personnes susceptibles de développer un SIDA, d'anticorps dirigés contre un HIV-2 ou d'un virus qui en est proche.

La détection peut être réalisée de toutes façons en soi connues. Elle peut comprendre une mise en contact de ces sondes soit avec les acides nucléiques obtenus à partir des cellules contenues dans ces sérums ou autres milieux biologiques, par exemple liquides céphalo-rachidiens, salives, etc... Elle peut aussi comprendre une mise en contact de ces sondes avec ces milieux eux-mêmes dès lors que leurs acides nucléiques ont été rendus accessibles à l'hybridation avec ces sondes, et ce dans des conditions permettant l'hybridation entre ces sondes et ces acides nucléiques. L'étape finale du diagnostic in vitro comprend alors la détection de l'hybridation éventuellement produite. Le susdit diagnostic mettant en jeu des réactions d'hybridation peut également être réalisé à l'aide de mélanges de sondes respectivement originaires d'un HIV-2 et d'un SIV-1 ou d'un HIV-1, d'un HIV-2 et d'un SIV, dès lors qu'il n'est pas nécessaire de faire une différence entre le type de virus recherché.

D'une façon générale, le procédé de diagnostic de la présence ou non du virus HIV-2 ou d'un variant dans des échantillons de sérums ou d'autres liquides ou tissus obtenus à partir de patients suspectés d'être porteurs du virus HIV-2 comprend les étapes suivantes :
1/ au moins une étape d'hybridation conduite dans des conditions stringentes, par mise en contact de l'ADN de cellules de l'échantillon du patient suspect avec l'une des susdites sondes marquées sur une membrane appropriée,
2/ le lavage de ladite membrane avec une solution assurant la conservation de ces conditions stringentes de l'hybridation,
3/ la détection de la présence ou non du virus HIV-2 par une méthode d'immunodétection.

Alternativement, l'hybridation précitée est conduite dans des conditions non stringentes et le lavage de la membrane est réalisé dans des conditions adaptées à celles de l'hybridation.

La demande décrit aussi les acides nucléiques correspondant à des séquences placées en des régions analogues de variants de SIV ainsi que tous les acides nucléiques dont les modifications résulteraient de la mise à profit de la dégénérescence du code génétique.

Les études comparatives qui ont aussi permis d'aboutir à des résultats relatifs aux protéines de noyau (core), ci-après dénommées "protéines gag" et aux protéines d'enveloppes, ci-après dénommées "protéines env", ont également été rapportés dans la demande de brevet européen n° 87/400.151.4, déjà citée. Ces résultats montrent que les protéines du noyau (protéines gag) dans HIV-2 présentent des différences moins accentuées par rapport à celles des virus HIV-1, que les protéines d'enveloppe (protéines env). Globalement les protéines env dans HIV-2 se sont révélées présenter des parentés immunologiques extrêmement faibles, sinon inexistentes, avec les protéines env correspondantes des virus HIV-1.

Au contraire des études comparatives effectuées entre les structures des séquences d'ADNc des virus HIV-2 et SIV permettent de mettre en évidence certaines caractéristiques communes qui apparaissent au niveau des protéines.

Globalement, les protéines de HIV-2 et de SIV-1 montrent des parentés immunologiques importantes.

La glycoprotéine majeure d'enveloppe de HIV-2 s'est révélée être plus proche immunologiquement de la glycoprotéine majeure d'enveloppe de SIV que de la glycoprotéine majeure d'enveloppe de HIV-1.

Ces constatations s'imposent non seulement au niveau des poids moléculaires : 130-140 kilodaltons pour les glycoprotéines majeures de HIV-2 et de SIV contre environ 110 pour la glycoprotéine majeure d'enveloppe de HIV-1, mais aussi au niveau des propriétés immunologiques, puisque des sérums prélevés à partir de malades infectés par HIV-2, et plus particulièrement des anticorps formés contre la gp140 de HIV-2 reconnaissent la gp140 de SIV-1mac, alors que dans des essais semblables les mêmes sérums et les mêmes anticorps de HIV-2 ne reconnaissent pas la gp110 de HIV-1. Mais les sérums anti-HIV-1 qui n'ont jamais réagi avec la gp140 de HIV-2 précipitent une protéine de 26 Kdal marquée par la ³⁵S-cystéine, contenue dans les extraits de HIV-2.

La protéine majeure du noyau (core) de HIV-2 semble présenter un poids moléculaire moyen (environ 26.000) intermédiaire entre celui de la p25 de HIV-1 et la p27 de SIV.

Ces observations résultent des essais réalisés avec des extraits viraux obtenus à partir du HIV-2 isolé à partir de l'un des patients susmentionnés. Des résultats similaires ont été obtenus avec des extraits viraux du HIV-2 isolé à partir du second patient.

Des études plus poussées ont conduit les inventeurs à reconnaître une première classe de peptides ayant des séquences d'aminoacides soit identiques, soit proches de séquences contenues à l'intérieur des structures des protéines gag et env de HIV-2 ou de SIV voire de HIV-1. Ces peptides sont notamment applicables au diagnostic d'une infection chez l'homme par le virus HIV-2 ou de l'un de ses variants.

L'invention a pour objet un peptide caractérisé par les propriétés suivantes :
- il est reconnu par des anticorps présents dans le sérum d'un patient infecté par un rétrovirus humain HIV-2, lesdit anticorps reconnaissant la glycoprotéine majeure d'enveloppe d'un rétrovirus HIV-2,
- il contient un nombre de résidus d'acides aminés n'excédant pas 40.

L'invention concerne également un peptide caractérisé par les propriétés suivantes :
- il est reconnu par des anticorps présents dans le sérum d'un patient infecté par un rétrovirus humain HIV-2, lesdits anticorps reconnaissant la glycoprotéine majeure d'enveloppe d'un rétrovirus HIV-2,
- il n'est pas reconnu par des anticorps présents dans le sérum d'un patient infecté par un rétrovirus humain HIV-1, lesdit anticorps reconnaissant la glycoprotéine majeure d'enveloppe d'un rétrovirus HIV-1,
- il contient un nombre de résidus d'acides aminés n'excédant pas 40.

A cet égard la présente invention concerne également des procédés et des compositions de diagnostic pour la détection in vitro d'anticorps dirigés contre un virus HIV-2 ou de ses variants, plus particulièrement dans des échantillons biologiques, notamment des sérums de patients ayant subi une infection par le virus HIV-2, certains de ces peptides permettant une discrimination particulièrement poussée entre les infections dues à des virus HIV-2 et à des virus HIV-1.

Ces études poussées ont également conduit à la possibilité de synthétiser des peptides immunogènes ou susceptibles d'être rendus immunogènes, présentant des caractéristiques de structures leur permettant d'induire in vivo la production d'anticorps susceptibles de reconnaître des protéines env à la fois dans HIV-1 et dans HIV-2 et, au moins pour certains de ces peptides, de se fixer tant sur des virus HIV-1 que sur des virus HIV-2, plus particulièrement aux fins de les neutraliser. L'utilisation de ces derniers types de peptides est donc particulièrement indiquée pour la production de principes actifs de vaccins contre les virus HIV, donc contre le SIDA.

Pour désigner ci-après les résidus d'aminoacides entrant dans la constitution des peptides selon l'invention, on aura recours, pour ceux des acides aminés ayant une signification univoque à la nomenclature internationale désignant chaque acide aminé naturel par une lettre unique (lettre majuscule) selon le tableau des correspondances qui suit :
- M: Méthionine
- L: Leucine
- I: Isoleucine
- V: Valine
- F: Phenylalanine
- S: Sérine
- P: Proline
- T: Thréonine
- A: Alanine
- Y: Tyrosine
- H: Histidine
- Q: Glutamine
- N: Asparagine
- K: Lysine
- D: Acide Aspartique
- E: Acide glutaminique
- C: Cystéine
- W: Tryptophane
- R: Arginine
- G: Glycine

Lorsqu'un acide aminé pourra, en raison de sa position au sein de la chaîne d'aminoacides caractéristique d'un peptide déterminé, prendre plusieurs significations, il pourra soit être désigné par un tiret "-", si sa signification peut être quelconque, soit par une lettre minuscule lorsque cet aminoacide pourra présenter un nombre limité de significations préférées, ce nombre étant cependant toujours supérieur à 1. Dans ce dernier cas, les significations possibles de cette lettre minuscule seront toujours précisées en rapport avec le peptide auquel il appartient.

Afin de faciliter la lecture, ces peptides seront désignés par une abréviation env ou gag suivie d'un indice numérique, par référence à des séquences d'aminoacides contenues, selon le cas, soit dans les protéines env soit dans les protéines gag de certains HIV-1, HIV-2 ou SIV. Il y sera encore fait référence dans ce qui suit.

Enfin dans les définitions qui suivent
- les groupes X représentent soit un groupe NH₂ libre ou amidé, notamment par un ou deux groupes alcoyle comprenant de 1 à 5 atomes de carbone, soit un groupe peptidique comprenant de 1 à 5 aminoacides, dont l'aminoacide N-terminal présente lui-même un groupe NH₂ libre ou amidé comme précédemment indiqué, et
- les groupes Z représentent, soit un groupe -OH libre ou alcoxyle et contenant alors un groupe alcoyle comprenant de 1 à 5 atomes de carbone, soit un groupe peptidique comprenant de 1 à 5 aminoacides, dont l'aminoacide C- terminal présente lui-même un groupe -OH libre ou alcoxyle, comme précédemment indiqué, les groupes de 1 à 5 acides aminés le cas échéant contenus dans X ou Z ou dans les deux à la fois étant tels, que leur présence n'est pas incompatible avec la préservation pour l'essentiel des propriétés immunologiques, le cas échéant immunogènes, des peptides qui en sont dépourvus.

Les peptides selon l'invention, qui ont en commun des propriétés immunologiques avec des antigènes de HIV-2 et, pour certains d'entre eux également avec des antigènes de HIV-1 ou de ses variants, sont caractérisés en ce qu'ils ont égaement une structure peptidique en commun avec les antigènes de SIV. De façon avantageuse, ces peptides comprennent normalement au plus 40 résidus d'acides aminés.

Des peptides préférés sont les suivants :
env1
   XRV-AIEKYL-DQA-LN-WGCAFRQVCZ
env2
   X-LE-AQI-QQEKNMYELQKLNZ
env3
   XELGDYKLVEITPIG-APT--KR-----Z
env4
   X----VTV-YGVP-WK-AT--LFCA-Z
env5
   X---QE--L-NVTE-F--W-NZ
env6
   XL---S-KPCVKLTPLCV--Z
env7
   X---N-S-IT--C-K----Z
env8
   X-I---YC-P-G-A-L-C-N-TZ
env9
   X------A-C------W--Z
env10
   X-G-DPE------NC-GEF-YCN-----NZ
env11
   X-----C-IKQ-I------G---YZ

Plus particulièrement l'invention concerne les peptides suivants :
env1
   XRV-AIEKYL-DQA-LN-WGCAFRQVCZ
env2
   X-LE-AQIQQEKNMYELQKLNZ
env3
   XELGDYKLVEITPIG-APT--KR-----Z
env4
   X----VTV-YGVP-W--AT--LFCA-Z
env5
   X----E--L-NVTE-F--W-NZ
env6
   XL---S-KPCVKL-PLC---Z
env7
   X---N-S-I---C-K----Z
env8
   X-I---YC-P-G-A-L-C-N-TZ
env9
   X------A-C------W--Z
env10
   X-G-DPE------NC-GEF-YC------NZ
env11
   X-----C-I-Q-I------G---YZ

Des peptides avantageux correspondant aux précédents, présentent les formules qui suivent :
env1
   XRVTAIEKYLQDQARLNSWGCAFRQVCZ, ou
   XRVTAIEKYLKDQAQLNAWGCAFRQVCZ
env2
   XSLEQAQIQQEKNMYELQKLNSWZ, ou
   XLLEEAQIQQEKNMYELQKLNSWZ
env3
   XELGDYKLVEITPIGFAPTKEKRYSSAHZ, ou
   XELGDYKLVEITPIGLAPTNVKRYTTG-Z
   (On remarquera que les peptides env1, env2, env3 attestent de la très grande parenté entre HIV-2 et SIV-1. En effet le premier peptide est inclu dans le génome de HIV-2 et le second, dans celui de SIV-1).
env4
   XabcdVTVeYGVPfWogAThiLFCAjZ,
   dans lesquels les lettres de a à j peuvent avoir les significations suivantes :
   a est C, E ou D
   b est T, K, D, N ou I
   c est Q ou L
   d est Y ou W
   e est F ou Y
   f est T, V ou A
   g est N ou E
   h est I ou T
   i est P ou T
   j est T ou S
   o est K ou R
env5
   XabcoEdeLfNVTEgFhiWjNZ,
   dans lequel les lettres de a à j peuvent avoir les significations suivantes :
   a est D ou P
   b est D ou N
   c est Y ou P
   d est I, V, I ou L
   e est T, V, E ou A
   f est V, G ou E ou -
   g est A, N, G ou S
   h est D ou N
   i est A ou M
   j est N, K ou E
   o est Q ou S
env6
   XLabcSdKPCVKLoPLCuefKZ,
   dans lequel les lettres de a à f peuvent avoir les significations suivantes :
   a est F ou W
   b est E ou D
   c est T ou Q
   d est I ou L
   e est A, S ou T
   f est M ou L
   o est T ou S
   u est V ou I
env7
   XabCNxSyIocdCeKfghiZ,
   dans lequel les lettres de a à i et x et y peuvent avoir les significations suivantes :
   a est N ou T ou I
   b est H ou S ou N
   c est E ou Q
   d est S, A ou C
   e est D ou P
   f est H, V ou D
   g est Y ou S
   h est W ou F
   i est D ou E
   x est T ou R
   y est V ou A
   o est T ou Q
env8
   XaIbcdYCxPeGfAgLhCiNjTZ,
   dans lequel les lettres de a à k et x peuvent avoir les significations suivantes :
   a est A ou P
   b est R ou P
   c est F, I ou C
   d est R ou H
   e est P ou A
   f est Y ou F
   g est L ou I
   h est R ou K
   i est - ou N
   j est D ou K
   x est A ou T
env9
   XwabcxyAdCefghizWjkZ,
   dans lequel les lettres de a à k et x à z peuvent avoir les significations suivantes :
   a est K ou - ou E
   b est R ou -
   c est P ou M ou I
   d est W ou H ou Y
   e est W ou N ou T ou R
   f est F ou I
   g est K ou S ou N ou G
   h est G ou R ou E
   i est - ou A ou T
   j est K ou N ou D ou S
   k est D ou A ou N ou K ou E
   w est N, D ou I
   x est R ou G ou K
   y est Q ou K ou R
   z est K ou E ou Q ou N
env10
   XaGbDPEcdefghNCiGEFjYCokxlmnNZ,
   dans lequel les lettres de a à n et x peuvent avoir les significations suivantes :
   a est K ou - ou G
   b est S ou G ou -
   c est V ou I
   d est A ou V ou T
   e est Y ou T ou M ou F
   f est M ou H
   g est W ou S
   h est T ou F
   i est R ou G
   j est L ou F
   o est N ou K
   k est M ou S
   l est W ou Q ou K ou G
   m est F ou L
   n est L ou F
   x est T ou S ou N
env11
   XabcdwCeIoQfIxgyhizGjklYZ,
   dans lequel les lettres de a à l et w à z peuvent avoir les significations suivantes :
   a est R ou T ou S ou N
   b est N ou I
   c est Y ou T
   d est A ou L ou V
   e est H ou R
   f est I ou F
   g est T ou M
   h est H ou Q ou A
   i est K ou E
   j est R ou K
   k est N ou A
   l est V ou M
   w est P ou Q
   x est N ou K
   y est W ou V
   z est V ou T ou K
   o est K ou R

La structure du peptide antigénique codé par le gène gag et désigné par gag1 est également représentée ci-après :
XDCKLVLKGLGaNPTLEEMLTAZ,
dans lequel la lettre a désigne M ou T.

Il sera remarqué que, d'une façon générale, les aminoacides ayant une signification univoque (donc représentés par une lettre majuscule correspondant à la nomenclature internationale) qui interviennent dans les définitions qui précèdent des peptides selon l'invention, se trouvent être la correspondance avec des aminoacides identiques placés dans le même ordre dans les séquences env ou gag correspondantes de la protéine env ou gag d'au moins l'un des HIV, ou de SIV-1.

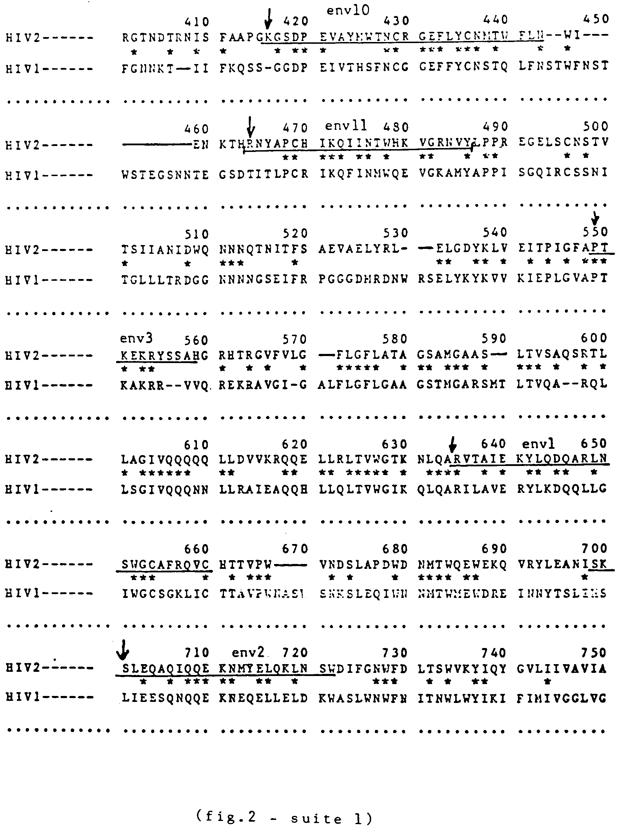

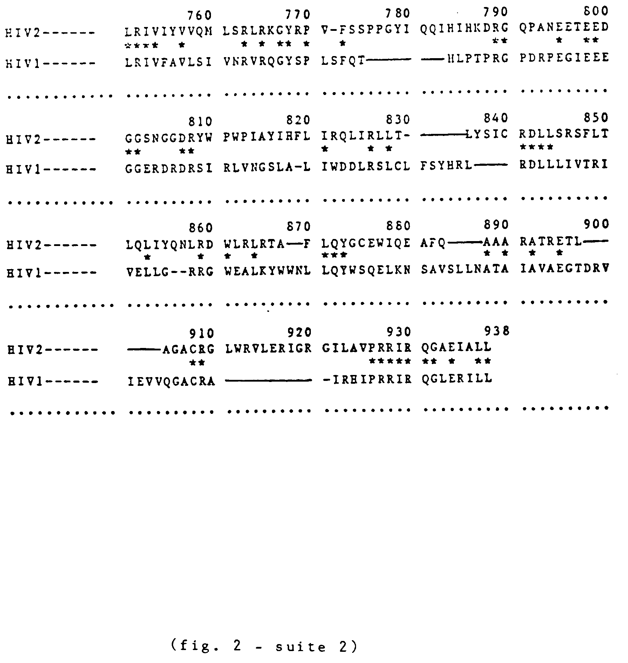

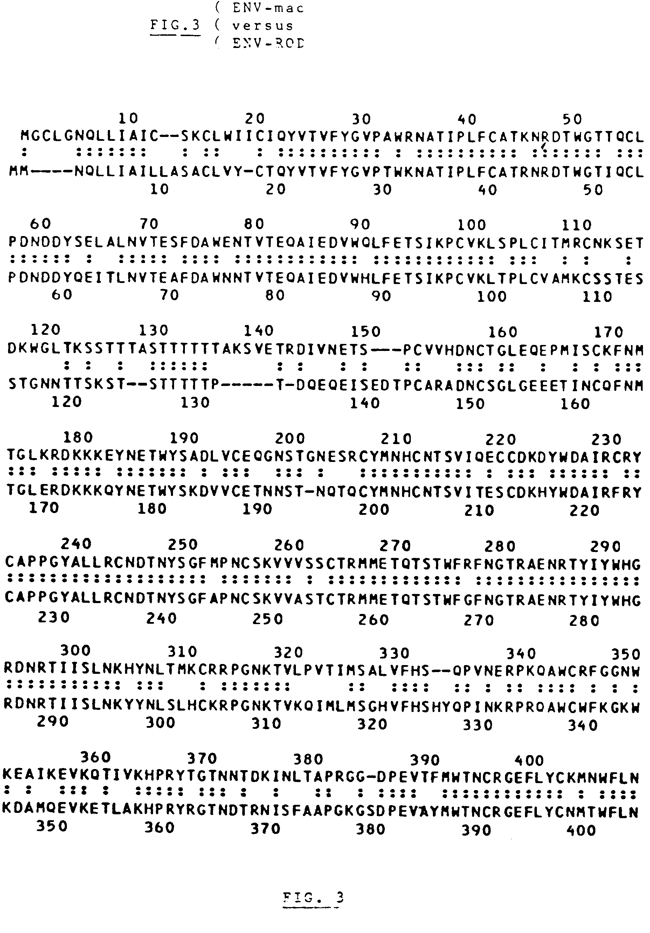

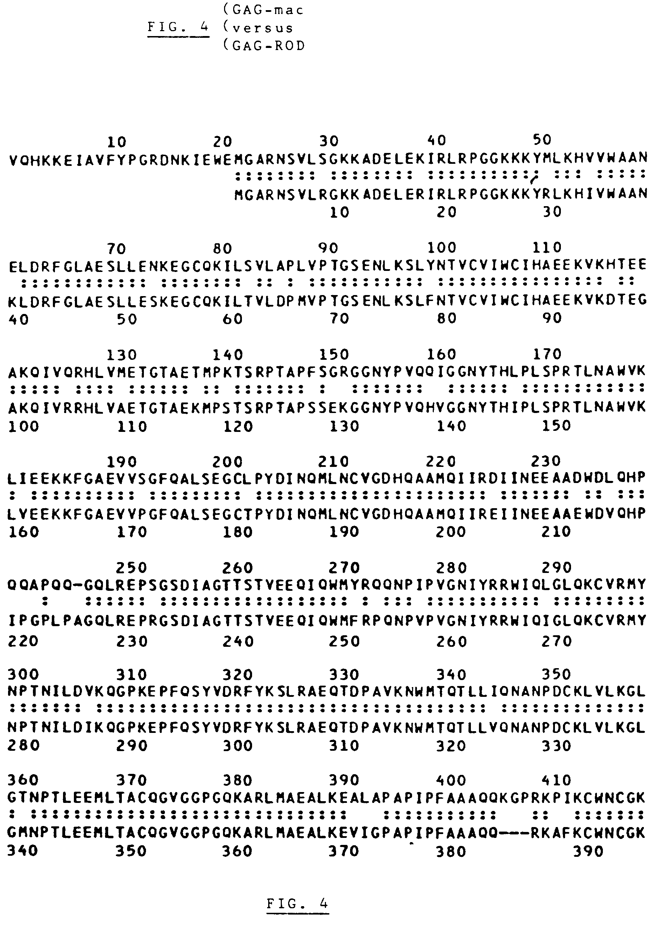

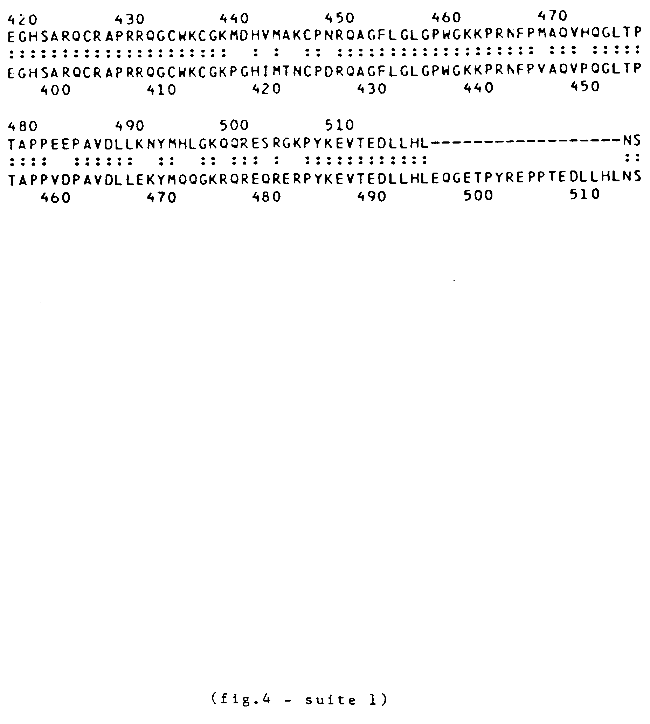

Les positions de ces séquences sont soulignées et repérées au sein des séquences d'aminoacides des protéines env respectivement de HIV-2 ROD (CNCM n° I-532) et HIV-1 BRU (CNCM n° I-232) représentées à la figure 2. Par ailleurs, les alignements des acides aminés des protéines env et gag respectivement de SIV-1mac (CNCM n° I.521) et de HIV-2 ROD sont présentées à la figure 3 et à la figure 4.

Les traits pleins qui apparaissent en certaines localisations de ces séquences visent à souligner que certains aminoacides contenus dans ces séquences ont été volontairement délétés au plan de la présentation, afin de permettre la mise en alignement d'aminoacides respectivement identiques (alors marqués d'un astérisque) ou de deux points verticaux sur une même ligne verticale dans les séquences des protéines correspondantes de HIV-1 et de HIV-2 d'une part, de SIV et de HIV-2 d'autre part.

Outre les peptides précités, l'invention concerne également les peptides modifiés par insertion et/ou délétion et/ou substitution d'un ou plusieurs acides aminés, pour autant que les propriétés antigéniques ou immunogènes desdits peptides ne sont pas modifiées, ou que les propriétés de reconnaissance de l'antigène ou de l'anticorps avec lesdits peptides ne sont pas substantiellement modifiées.

Dans un mode de réalisation particulièrement préféré, l'invention concerne des peptides ayant des propriétés immunologiques en commun avec l'ossature peptidique de la glycoprotéine d'enveloppe des virus de la classe HIV-2, ces peptides contenant un nombre de résidus d'acides aminés n'excédant pas 40.

Ces peptides préférés selon l'invention ont les séquences suivantes :

env1
RVTAIEKYLQDQARLNSWGCAFRQVC
AIEKYLQDQ
RVSAIEKYLKDQAQLNAWGCAFRQVC
AIEKYLKDQ
env2
SLEQAQIQQEKNMYELQKLNSW
QIQQEKN
LLEEAQIQQEKNMYELQKLNSW
env3
ELGDYKLVEITPIGFAPTKEKRYSSAH
YKLVEITPIGFAPTKEK
ELGDYKLVEITPIGLAPTNVKRYTTG-
YKLVEITPIGLAPTNVK
env4
CTQYVTVFYGVPTWKNATIPLFCAT
VTVFYGVPTWKNAT
CIQYVTVFYGVPAWRNATIPLFCAT
VTVFYGVPAWRNAT

EKLWVTVYYGVPVWKEATTTLFCAS
     VTVYYGVPVWKEAT
EDLWVTVYYGVPVWKEATTTLFCAS
     VTVYYGVPVWKEAT
DNLWVTVYYGVPVWKEATTTLFCAS
     VTVYYGVPVWKEAT
env5
DDYQEITL-NVTEAFDAWNN
        L-NVTEAF
DDYSELAL-NVTESFDAWEN
        L-NVTESF
PNPQEVVLVNVTENFNMWKN
        LVNVTENF
PNPQEIELENVTEGFNMWKN
        LENVTEGF
PNPQEIALENVTENFNMWKN
        LENVTENF
env6
ETSIKPCVKLTPLCVAMK
ETSIKPCVKLSPLCITMR
DQSLKPCVKLTPLCVSLK
DQSLKPCVKLTPLCVTLN
      PCVKLTPLCV
env7
NHCNTSVITESCD
   NTSVIT
NHCNTSVIQECCD
   NTSVIQ
TSCNTSVITQACP
   NTSVIT
INCNTSVITQACP
   NTSVIT
INCNTSAITQACP
   NTSAIT

env8
YCAPPGYALLRC-NDT
YCAPAGFAILKCNNKT
YCAPAGFAILKCNDKK
YCAPAGFAILKCRDKK
env9
NKRPRQAWCWFKG-KWKD
NERPKQAWCRFGG-NWKE
N--MRQAHCNISRAKWNA
D--IRRAYCTINETEWDK
I--IGQAHCNISRAQWSK
env10
KGSDPEVAYMWTNCRGEFLYCNMTWFLN
NCRGEFLYCN
GG-DPEVTFMWTNCRGEFLYCKMNWFLN
NCRGEFLYCK
-GGDPEIVTHSFNCGGEFFYCNSTQLFN
NCGGEFFYCN
-GGDPEITTHSFNCRGEFFYCNTSKLFN
NCRGEFFYCN
-GGDPEITTHSFNCGGEFFYCNTSGLFN
NCGGEFFYCN
env11
RNYAPCHIKQIINTWHKVGRNVY
CHIKQII
RNYVPCHIRQIINTWHKVGKNVY
CHIRQII
TITLPCRIKQFINMWQEVGKAMY
CRIKQFI
SITLPCRIKQIINMWQKTCKAMY
CRIKQII
NITLQCRIKQIIKMVAGR-KAIY
CRIKQII
gag1
DCKLVLKGLGTNPTLEEMLTA

Les peptides selon l'invention peuvent encore avantageusement être préparés par les techniques classiques, dans le domaine de la synthèse des peptides. Cette synthèse peut être réalisée en solution homogène ou en phase solide.

Par exemple, on aura recours à la technique de synthèse en solution homogène décrit par HOUBENWEYL dans l'ouvrage intitulé "Méthode der Organischen Chemie" (Méthode de la Chimie Organique) édité par E. Wunsch, vol. 15-I et II., THIEME, Stuttgart 1974.

Cette méthode de synthèse consiste à condenser successivement deux-à-deux les aminoacyles successifs dans l'ordre requis, ou à condenser des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amines de l'un et carboxyles de l'autre ou vice-versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthyl-amino-propyl)-carbodiimide. Lorsque l'aminoacyle mis en oeuvre possède une fonction acide supplémentaire (notamment dans le cas de l'acide glutamique), ces fonctions seront par exemple protégées, par des groupes t-bustylester.

Dans le cas de la synthèse progressive, acide aminé par acide aminé, la synthèse débute de préférence par la condensation de l'amino-acide C-terminal avec l'aminoacide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptide synthesis" (J. Am. Soc., 45, 2149-2154).

Pour fabriquer une chaîne peptidique selon le procédé de MERRIFIELD, on a recours à une résine polymère très poreuse, sur laquelle on fixe le premier acide aminé C-terminal de la chaîne. Cet acide aminé est fixé sur la résine par l'intermédiaire de son groupe carboxylique et sa fonction amine est protégée, par exemple par le groupe t-butyloxycarbonyle.

Lorsque le premier acide aminé C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième acide aminé qui fournit le second amino-acyle de la séquence recherché, à partir du résidu amino-acyle C-terminal sur la fonction amine déprotégée du premier acide aminé C-terminal fixé sur la chaîne. De péférence, la fonction carboxyle de ce deuxième acide aminé est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux acide aminés, et dont la fonction amine terminale est protégée. Comme précédemment, on déprotège la fonction amine et on peut ensuite procéder à la fixation du troisième aminoacyle, dans les conditions analogues à celles de l'addition du deuxième acide aminé C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents acide aminés constituant la chaîne peptidique et on détache le peptide de la résine par exemple à l'aide d'acide fluorydrique.

L'invention concerne également les oligomères hydrosolubles des peptides monomères sus-indiqués. L'oligomérisation peut provoquer un accroissement de l'immunogénicité des peptides monomères selon l'invention. Sans qu'une telle indication chiffrée puise être considérée comme limitative, on mentionnera néanmoins que ces oligomères peuvent, par exemple, contenir de 2 à 10 unités monomères.

Les unités monomères entrant dans cet oligomère sont soit toutes constituées par le polypeptide de séquence 1 ou par le polypeptide de séquence 2, soit par l'un et l'autre de ces polypeptides.

On peut avoir recours, pour réaliser l'oligomérisation, à toute technique de polymérisation couramment utilisée dans le domaine des peptides, cette polymérisation étant conduite jusqu'à l'obtention d'un oligomère ou polymère contenant le nombre de motifs monomères requis pour l'acquisition de l'immunogénicité désirée.

Une méthode d'oligomérisation ou de polymérisation du monomère consiste dans la réaction de celui-ci avec un agent de réticulation tel que le glutaraldéhyde.

On peut également avoir recours à d'autres méthodes d'oligomérisation ou de couplage, par exemple à celle mettant en jeu des couplages successifs d'unités monomères, par l'intermédiaire de leurs fonctions terminales carboxyle et amine en présence d'agents de couplage homo- ou hétéro- bifonctionnels.

On peut également pour la production de molécules comportant un ou plusieurs motifs de 17 acides aminés tels que définis ci-dessus, avoir recours à des techniques du génie génétique mettant en oeuvre des micro-organismes transformés par un acide nucléique déterminé comprenant des séquences nucléotidiques appropriées correspondantes.

Les acides nucléiques dècrits dans la demande contiennent une ou plusieurs séquences issues de la séquence de l'ADNc du virus HIV-2 ROD. Ces séquences repérées par la numérotation figurant sur la séquence précédemment décrite, codent pour certains peptides intéressants de l'invention.
Séquence codant pour env1 nucléotides 7850 à 7927
Séquence codant pour env2 nucléotides 8030 à 8095
Séquence codant pour env3 nucléotides 7601 à 7636
Séquence codant pour env4 nucléotides 6170 à 6247
Séquence codant pour env5 nucléotides 6294 à 6349
Séquence codant pour env6 nucléotides 6392 à 6445
Séquence codant pour env7 nucléotides 6724 à 6763
Séquence codant pour env8 nucléotides 6794 à 6838
Séquence codant pour env9 nucléotides 7112 à 7162
Séquence codant pour env10 nucléotides 7253 à 7336
Séquence codant pour env11 nucléotides 7358 à 7426
Séquence codant pour gag1 nucléotides 1535 à 1597

les acides nucléiques correspondants du virus SIV décrits contiennent, une ou plusieurs séquences issues de l'ADNc du virus SIV-1. Ces séquences codant pour les peptides env1 à env11 et gag1 peuvent être repérés sur la figure 3 par comparaison avec les séquences correspondantes décrites pour HIV-2.

La demande dècrit aussi les acides nucléiques correspondant à des séquences placées en des régions analogues des ADNc dérivés de variants de HIV-2 ROD ou de SIV, ainsi que tous les acides nucléiques dont les modifications vis à vis des précédents résulteraient de la mise à profit de la dégénérescence du code génétique.

L'invention concerne encore les conjugués obtenus par couplage covalent des peptides selon l'invention (ou des susdits oligomères) à des molécules porteuses (naturelles ou synthétiques), physiologiquement acceptables et non toxiques, par l'intermédiaire de groupements réactifs complémentaires respectivement portés par la molécule porteuse et le peptide. Des exemples de groupements appropriés sont illustrés dans ce qui suit :

A titre d'exemple de molécules porteuses ou supports macromoléculaires entrant dans la constitution des conjugués selon l'invention, on mentionnera des protéines naturelles, telles que l'anatoxine tétanique, l'ovalbulmine, des sérums albumines, des hémocyamines, etc...

A titre de support macromoléculaires synthétiques, on mentionnera par exemple des polylysines ou des poly(D-L-alanine)-poly(L-lysine).

La littérature mentionne d'autres types de supports macromoléculaires susceptibles d'être utilisés, lesquels présentent en général un poids moléculaire supérieur à 20 000.

Pour synthétiser les conjugués selon l'invention, on peut avoir recours à des procédés connus en soi, tels que celui décrit par FRANTZ et ROBERTSON dans Infect. and Immunity, 33, 193-198 (1981), ou celui décrit dans Applied and Environmental Microbiology, (octobre 1981), vol. 42, n° 4, 611-614 par P.E. KAUFFMAN en utilisant le peptide et la molécule porteuse appropriée.

Dans la pratique, on utilisera avantageusement comme agent de couplage les composés suivants, cités à titre non limitatif : aldéhyde glutarique, chloroformiate d'éthyle, carbodiimides hydrosolubles [N-éthyl-N'(3-diméthylamino-propyl) carbodiimide, HCl], diisocyanates, bis-diazobenzidine, di- et trichloro-s-triazines, bromures de cyanogène, ainsi que les agents de couplage mentionnés dans Scand. J. Immunol., (1978), vol. 8, p. 7-23 (AVRAMEAS, TERNYNCK, GUESDON).

On peut avoir recours à tout procédé de couplage faisant intervenir d'une part une ou plusieurs fonctions réactives du peptide et d'autre part, une ou plusieurs fonctions réactives de molécules supports. Avantageusement, il s'agit des fonctions carboxyle et amine, lesquelles peuvent donner lieu à une réaction de couplage en présence d'un agent de couplage du genre de ceux utilisés dans la synthèse des protéines, par exemple, le 1-éthyl-3-(3-diméthylaminopropyl)-carbodiimide, le N-hydroxybenzotriazole, etc... On peut encore avoir recours à la glutaraldéhyde, notamment lorsqu'il s'agit de relier entre eux des groupes aminés respectivement portés par le peptide et la molécule support.

Les peptides selon l'invention possèdent des propriétés antigéniques. Ils peuvent donc être utilisés dans des procédés de diagnostic pour la détection d'une infection par le virus HIV-2.

Comme on l'a déjà mentionné, des études ont permis de distinguer deux groupes de peptides pouvant être mis en oeuvre dans des procédés de détection d'anticorps contre le virus HIV-2 dans un fluide biologique humain, notamment un sérum ou un liquide céphalo-rachidien.

Un premier groupe (I) comprend les peptides gag₁. Ces peptides reconnaissent des anticorps anti-HIV-2 et sont donc capables de détecter une infection par HIV-2. Ils reconnaissent également dans une certaine mesure des anticorps anti-HIV-1.

Un second groupe (II) comprend des peptides qui correspondent plus particulièrement à ceux qui sont situés dans la partie transmembranaire et dans la fin de la partie externe de la protéine d'enveloppe. Ces peptides sont ceux précédemment désignés par env1, env2 et env3. Ils permettent la reconnaissance spécifique de la présence d'anticorps contre HIV-2 et permettent donc de discriminer chez une personne les infections passées ou présentes dues à un HIV, plus particulièrement entre celles qui ont été provoquées par un HIV-2 et celles qui l'ont été par un HIV-1.

L'invention concerne également une composition contenant au moins l'un des susdits peptides ou au moins un oligomère de ce peptide, caractérisée en ce qu'elle a la capacité d'être reconnue par des sérums d'origine humaine contenant des anticorps contre le virus HIV-2.

L'invention concerne un procédé de diagnostic in vitro un ou des peptides selon l'invention pour la détection d'anticorps contre HIV-2 dans des fluides biologiques, en particulier dans des sérums humains.

D'une façon générale le procédé de diagnostic in vitro ci-dessus comprend les étapes suivantes :
- la mise en contact de ce liquide biologique avec lesdits peptides,
- la détection de la présence éventuelle d'un complexe peptide-anticorps par des méthodes physiques ou chimiques, dans ledit liquide biologique.

Dans un mode de réalisation préféré de l'invention, la détection du complexe antigène-anticorps est réalisée grâce à des tests immunoenzymatiques (du type ELISA), immunofluorescents (du type IFA), radioimmunologiques (du type RIA) ou des tests de radioimmunoprécipitation (du type RIPA).
Ainsi l'invention concerne également tout peptide selon l'invention marqué à l'aide d'un marqueur adéquat du type enzymatique, fluorescent, radioactif, etc...

De telles méthodes comprennent par exemple les étapes suivantes :
- dépôt de quantités déterminées d'une composition peptidique selon l'invention dans les puits d'une microplaque de titration,
- introduction dans lesdits puits de dilutions croissantes du sérum devant être diagnostiqué,
- incubation de la microplaque,
- rinçages répétés de la microplaque,
- introduction dans les puits de la microplaque d'anticorps marqués contre des immunoglobulines du sang, le marquage de ces anticorps ayant été réalisé à l'aide d'une enzyme sélectionnée parmi celles qui sont capables d'hydrolyser un substrat en modifiant l'absorption des radiations de ce dernier, au moins à une longueur d'onde déterminée,
- détection, en comparaison avec un témoin de contrôle, de la quantité de substrat hydrolysé.

L'invention concerne également des coffrets ou kits pour le diagnostic in vitro de la présence d'anticorps contre les virus HIV-2 et, dans certains cas, HIV-1 dans un milieu biologique qui comprennent :
- une composition peptidique selon l'invention,
- les réactifs pour la constitution du milieu propice à la réalisation de la réaction immunologique,
- les réactifs permettant la détection du complexe antigènes-anticorps produit par la réaction immunologique. De tels réactifs peuvent également porter un marqueur, ou être susceptibles d'être reconnus à leur tour par un réactif marqué. Plus particulièrement dans le cas où la composition polypeptidique sus-mentionnée n'est pas marquée.
- un tissu fluide biologique de référence dépourvu d'anticorps reconnus par la composition polypeptidique sus-mentionnée,

L'invention concerne les anticorps eux-mêmes formés contre les peptides de l'invention.

Il va de soi que cette production n'est pas limitée aux anticorps polyclonaux.

Elle s'applique encore à tout anticorps monoclonal produit par tout hybridome susceptible d'être formé, par des méthodes classiques, à partir des cellules spléniques d'un animal, notamment de souris ou de rat, immunisés contre l'un des peptides de l'invention, d'une part et des cellules d'une lignée de cellule myélome approprié d'autre part, et d'être sélectionné, par sa capacité à produire des anticorps monoclonaux reconnaissant le peptide initialement mis en oeuvre pour l'immunisation des animaux.

L'invention concerne également des compositions immunogènes pour la production de vaccins dont le principe actif est constitué par au moins un peptide selon l'invention, ou un oligomère de ce peptide, ou un peptide sous forme conjuguée avec une molécule porteuse, caractérisées en ce qu'elles induisent la production d'anticorps contre les susdits peptides en quantité suffisante pour aussi inhiber les protéines du rétrovirus HIV-2, voire même le rétrovirus HIV-2 entrant en association avec un véhicule pharmaceutiquement acceptable.

Les compositions immunogènes pour la production de vaccins comprennent de façon avantageuse plus particulièrement au moins l'un des peptides précédemment désignés par env4, env5, env6, env7, env8, env9, env10, env11 voir des mélanges de ceux-ci.

Parmi ces peptides aptes à constituer des principes actifs de vaccins certains sont particulièrement préférés car ils possèdent une structure de base en acides aminés correspondant à des régions des glycoprotéines d'enveloppe qui présentent un important degré de conservation, non seulement dans les HIV-2, et dans les SIV, mais également dans les HIV-1. Ces peptides particulièrement préférés sont les peptides désignés par env4, certains peptides env5, env6 et env10.

Dans un mode de réalisation préféré de l'invention les peptides immunogènes (ou fragments de ces peptides) aptes à constituer des principes actifs de vaccins sont choisis parmi ceux dont les formules correspondent à des séquences qui, dans les glycoprotéines d'enveloppe de HIV-2, SIV et HIV-1 présentant une homologie en acides aminés supérieure à 50%, qui appartiennent à la partie externe de l'enveloppe du virus, qui sont dépourvus ou presque de délétions, et qui renferment des résidus de cystéine favorables à la stabilisation des liaisons et à la constitution de boucles d'ancrage.

Les peptides suivants appartiennent à cette catégorie de peptides préférés.
env4
   XVTV-YGVP-W--ATZ
env5
   XL-NVTE-FZ
env6
   XKPCVKL-PLC-Z
env7
   XN-S-I-Z
env10
   XNC-GEF-YC-Z
env11
   XC-I-Q-IZ

Des compositions pharmaceutiques avantageuses sont constituées par des solutions, suspensions ou liposomes injectables contenant une dose efficace d'au moins un produit selon l'invention. De préférence, ces solutions, suspensions ou liposomes sont réalisés dans une phase aqueuse stérilisée isotonique, de préférence saline ou glucosée.

L'invention concerne plus particulièrement de telles suspensions, solutions ou forme liposome qui sont aptes à être administrées par injections intradermiques, intramusculaires ou sous-cutanées, ou encore par scarifications.

Elle concerne également des compositions pharmaceutiques administrables par d'autres voies, notamment par voie orale.

Les compositions pharmaceutiques selon l'invention, utilisables en tant que vaccins pour être efficaces dans la production d'anticorps contre le virus HIV-2, peuvent à titre d'exemple être administrées à des doses situées entre 10 et 500 µg/kg, de peptides selon l'invention, de préférence de 50 à 100 µg/kg.

Ces doses sont citées à titre d'exemple et ne possèdent en aucun cas un caractère limitatif.

Comme on l'a déjà indiqué plus haut les différents peptides qui ont été définis peuvent comprendre des modifications qui n'ont pas pour effet de modifier de façon fondamentale leurs propriétés immunologiques. Les peptides équivalents qui en résultent entrent dans le champ des revendications qui suivent. A titre d'exemples de peptides équivalents on mentionnera ceux dont les structures en correspondance avec des régions des ADNc d'autres variants de HIV-2 de SIV ou de HIV-1, lorsque ces régions ont été mises en alignement dans des conditions semblables à celles qui ont été évoquées ci-dessus, à propos de HIV-2 ROD, SIV et HIV-1 BRU. A titre d'autres de ces peptides, on mentionnera ceux dont les structures sont en correspondance avec de telles régions dans les ADNc qui ont fait l'objet de dépôts à la CNCM, notamment sous les numéros I-502, I-642 (HIV-2 IRMO), I-643 (HIV-2 EHO) ainsi que, dans les cas appropriés, des variants de HIV-1 qui ont fait l'objet de dépôts à la CNCM sous les numéros I-232, I-240, I-241 I-550, I-551.

Les peptides selon l'invention peuvent encore être définis par les formules suivantes (dans lesquels X, Z et les tirets "-" ont les significations sus-indiquées) :

XRV-AIEKYL-DQA-LN-WGCAFRQVCZ
XAIEKYL-DZ

X-LE-AQIQQEKNMYELQKLNSWZ
XQIQQEKNZ

XELGDYKLVEITPIG-APT--KR-----Z
XYKLVEITPIG-APT--KRZ

X----VTV-YGVP-W--AT--LFCA-Z
XVTV-YGVP-W--ATZ

X----E--L-NVTE-F--W-NZ
XL-NVTE-FZ

XL---S-KPCVKL-PLC----Z
XKPCVKL-PLC-Z
XS-KPCVKL-PLC-Z

X---N-S-I---C-Z
XN-S-I-Z

XYC-P-G-A-L-C-N-TZ

X------A-C------W--Z

NKRPRQAWCWFKG-KWKD

X-G-DPE------NC-GEF-YC------NZ

X-----C-I-Q-I------G---YZ

L'invention concerne également outre les peptides de SIV déjà décrits, les protéines codées par l'ADNc du virus SIV. Elle concerne également les protéines de tout virus immunologiquement étroitement apparenté à SIV-1mac, en particulier tout virus dont les protéines et les glycoprotéines d'enveloppe croisent immunologiquement et dont les ADNc présentent un pourcentage d'homologie d'au moins 95% et de préférence d'au moins 98%.

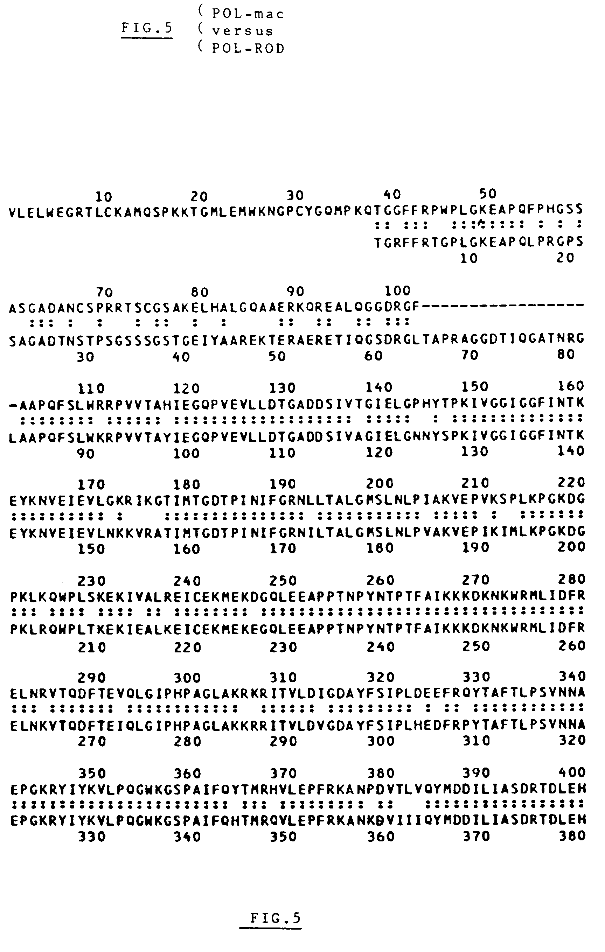

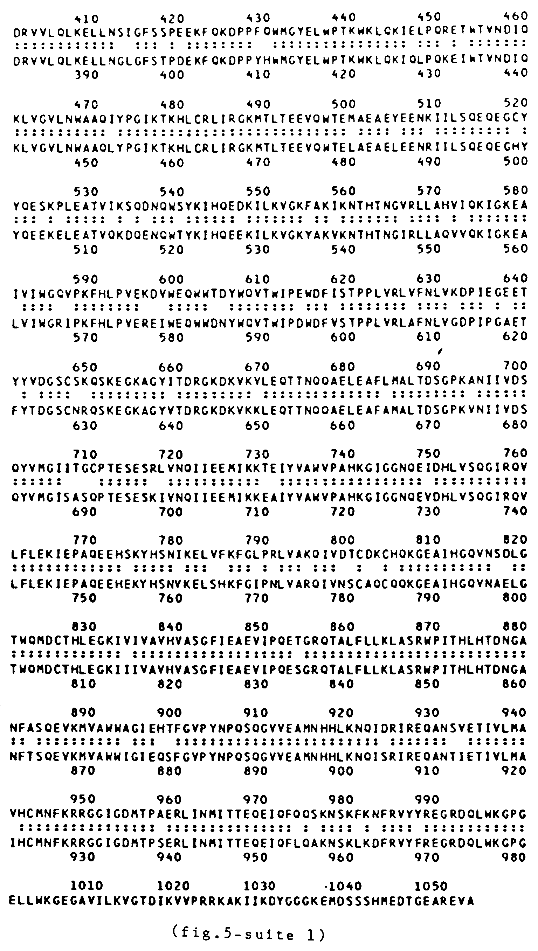

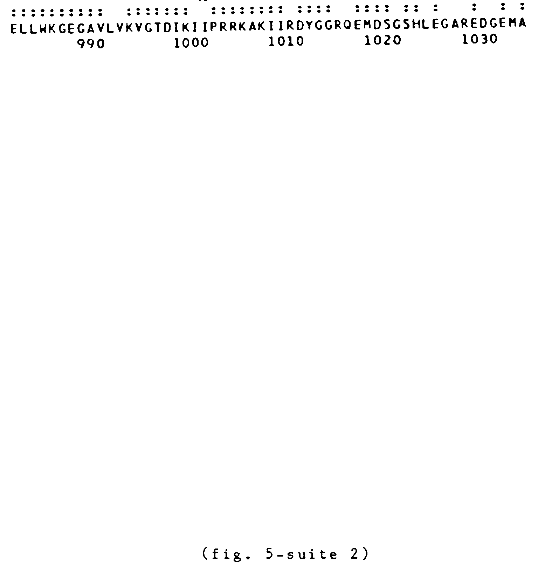

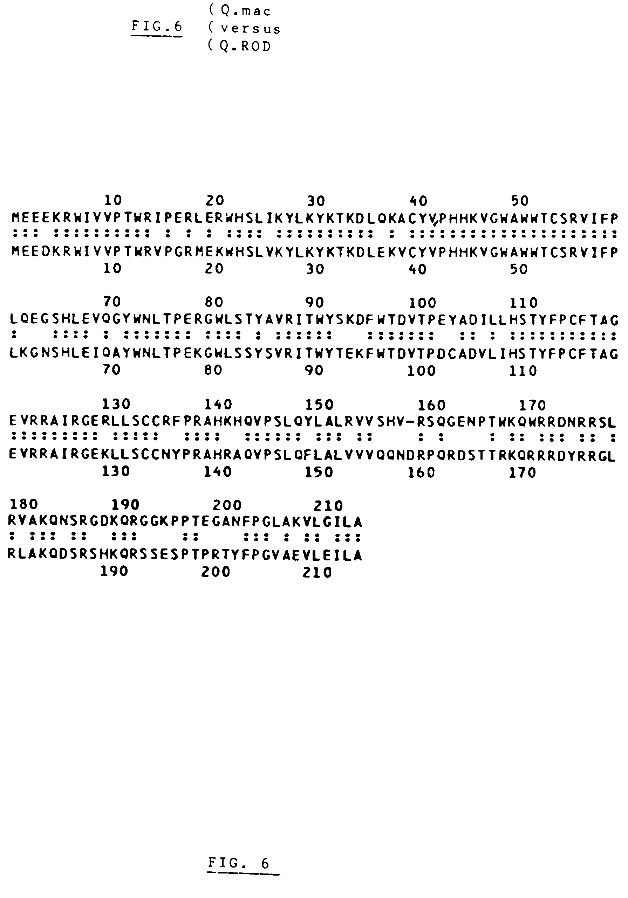

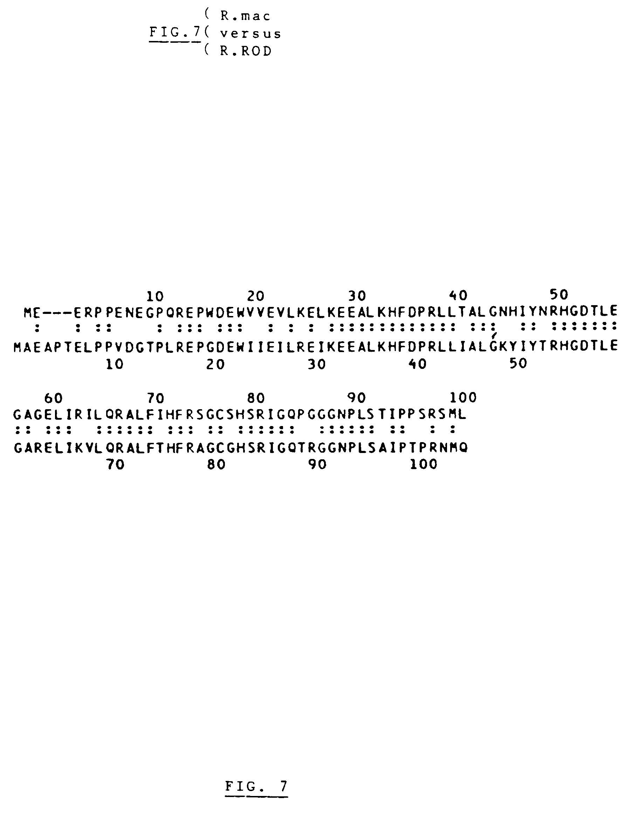

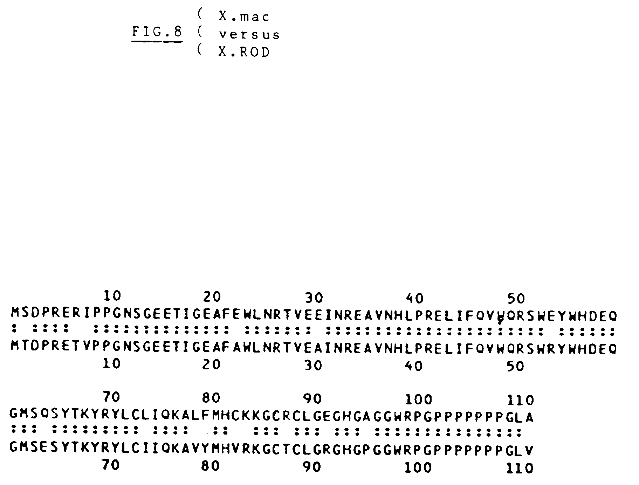

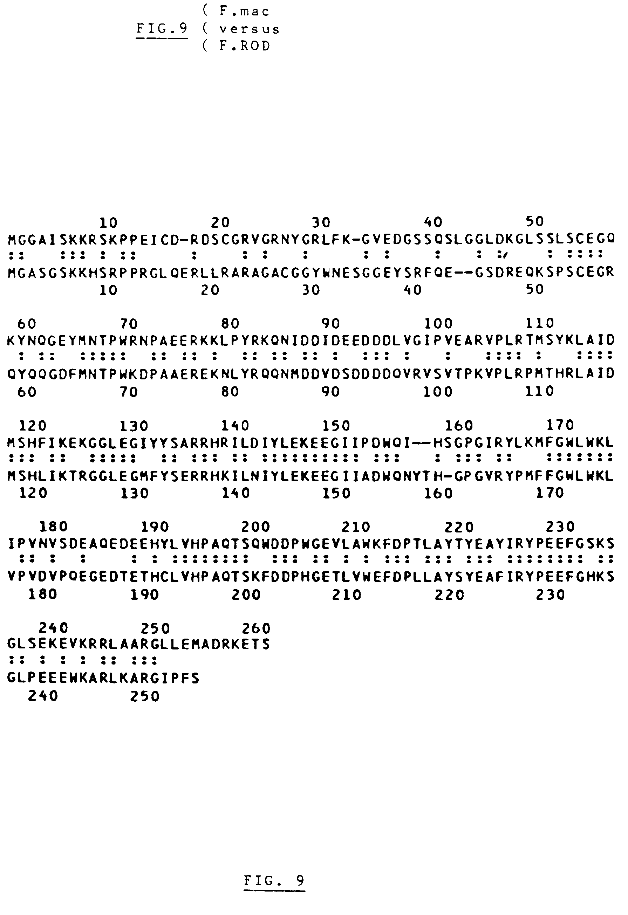

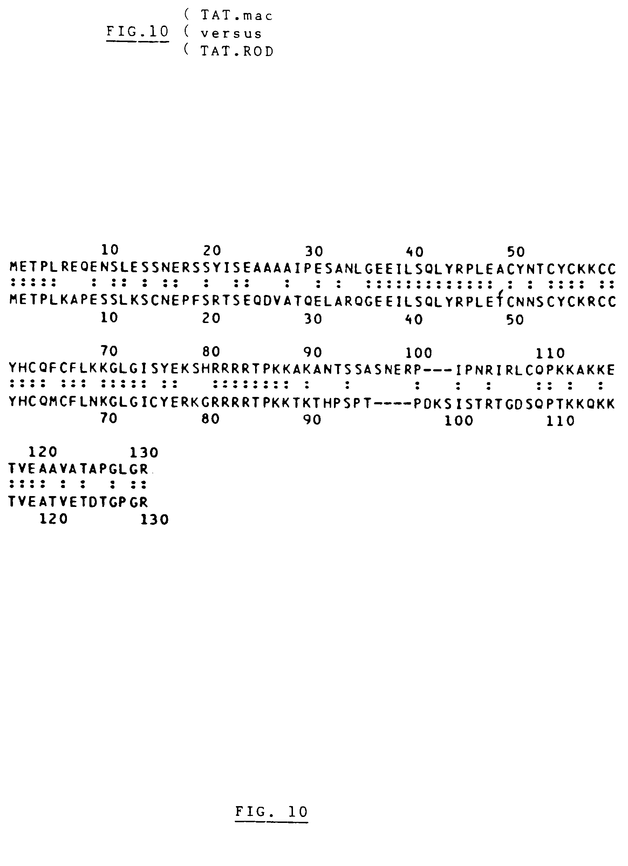

En particulier l'invention concerne :
1/ les protéines et glycoprotéines de l'enveloppe codées par le gène env et représentées à la figure 3,
2/ la protéine GAG représentée à la figure 4,
3/ la protéine POL représentée à la figure 5,
4/ la protéine Q représentée à la figure 6,
5/ la protéine R représentée à la figure 7,
6/ la protéine X représentée à la figure 8,
7/ la protéine F représentée à la figure 9,
8/ la protéine TAT représentée à la figure 10,

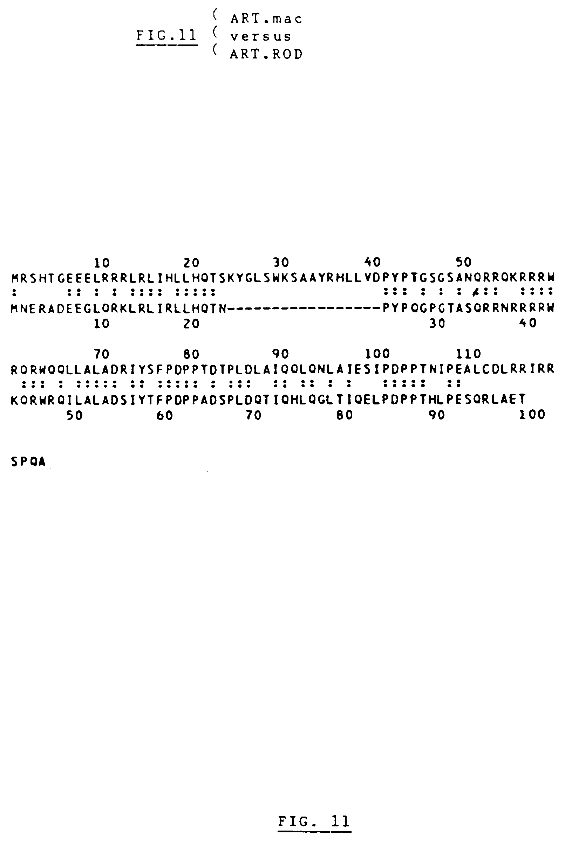

L'invention concerne notamment un peptide reconnaissant des anticorps dirigés contre des protéines de HIV-2 caractérisé en ce qu'il comprend tout ou partie des séquences d'acides aminés choisies parmi les séquences suivantes :
POL_{ROD} ou POL_{mac} représenté à la figure 5
Q_{ROD} ou Q_{mac} représenté à la figure 6
R_{ROD} ou R_{mac} représenté à la figure 7
X_{ROD} ou X_{mac} représenté à la figure 8
F_{ROD} ou F_{mac} représenté à la figure 9
TAT_{ROD} ou TAT_{mac} représenté à la figure 10
ART_{ROD} ou ART_{mac} représenté à la figure 11.

Les acides aminés des protéines précitées de SIV, ont été représentés en alignement avec les séquences d'acides aminés des protéines correspondantes du virus HIV-2 ; les points verticaux figurant entre les deux séquences correspondent aux acides aminés communs entre les protéines des deux virus.

Les séquences d'ADNc codant pour les protéines précitées apparaissent sur la figure 1B. Les séquences nucléiques précitées peuvent être modifièes dès lors que la séquence nucléique modifiée, code également pour les protéines du rétrovirus SIV ou d'un variant.

Ces séquences d'ADNc repérées par la numérotation figurant sur les séquences décrites précédemment (figure 1B) sont les suivantes :
- séquence codant pour GAG, nucléotides 551 à 2068
- séquence codant pour POL, nucléotides 1726 à 4893
- séquence codant pour Q, nucléotides 4826 à 5467
- séquence codant pour X, nucléotides 5298 à 5633
- séquence codant pour R, nucléotides 5637 à 5939
- séquence codant pour F, nucléotides 8569 à 9354
- séquence codant pour TAT-1 nucléotides 5788 à 6084
- séquence codant pour ART-1 nucléotides 6014 à 6130
- séquence codant pour TAT-2 nucléotides 8296 à 8391
- séquence codant pour ART-2 nucléotides 8294 à 8548
- séquence codant pour ENV nucléotides 6090 à 8732

L'invention concerne donc naturellement les protéines précédemment décrites, lorsqu'elles sont obtenues à partir du virus SIV ou lorsqu'elles sont préparées par une méthode de synthèse, notamment par l'une des méthodes déjà citées en rapport avec la synthèse des peptides de plus petite taille.

L'invention concerne également l'utilisation des protéines précédentes pour le diagnostic de la présence éventuelle d'anticorps dirigés contre les protéines de HIV-2, voire contre HIV-2 en entier, ou pour certaines d'entre elles l'utilisation aux fins de diagnostic d'une infection due à l'un des virus HIV. Ainsi le peptide GAG codé par le gène correspondant peut être utilisé pour repérer la présence éventuelle d'anticorps anti-HIV-1 ou anti-HIV-2. Les protéines ENV sont utilisées de préférence pour le diagnostic spécifique d'une infection due à HIV-2 ou un de ses variants, parfois pour le diagnostic d'une infection par HIV-2 ou HIV-1.

L'invention concerne donc également un procédé de diagnostic in vitro de détection d'anticorps contre HIV-2 et éventuellement contre HIV-1 dans des fluides biologiques et en particulier dans des sérums humains. De tels procédés applicables pour l'utilisation des protéines précédentes de SIV comme protéines de diagnostic, ont déjà été décrits dans la présente invention.

L'invention concerne aussi des coffrets ou "kits" pour le diagnostic in vitro de la présence d'anticorps le virus HIV-2 et dans certains cas contre HIV-1 dans un milieu biologique. De tels kits mettant en oeuvre les peptides précédents ont également été décrits dans la présente invention.

L'invention concerne également des compositions immunogènes pour la production de vaccins, dont le principe actif est constitué de façon avantageuse par au moins la partie de la protéine ENV du virus SIV, cette protéine pouvant être sous forme conjuguée avec une molécule porteuse. Ces compositions immunogènes induisent la production d'anticorps contre le susdit peptide en quantité suffisante pour inhiber les protéines du rétrovirus HIV-2, voire le rétrovirus HIV-2 lui-même.

Toutefois l'utilisation aux fins de diagnostic des protéines de SIV n'est en rien limitée à celle des seuls protéines ENV ou GAG. D'autres protéines parmi celles décrites peuvent être envisagées, pour préparer des compositions de diagnostic voire de vaccin.

## Revendications

1. Peptide caractérisé par les propriétés suivantes :
- il est reconnu par des anticorps présents dans le sérum d'un patient infecté par un rétrovirus humain HIV-2, lesdits anticorps reconnaissant la glycoprotéine majeure d'enveloppe d'un rétrovirus HIV-2,
- il contient un nombre de résidus d'acides aminés n'excédant pas 40.

2. Peptide selon la revendication 1, caractérisé par les propriétés suivantes :
- il est reconnu par des anticorps présents dans le sérum d'un patient infecté par un rétrovirus humain HIV-2, lesdits anticorps reconnaissant la glycoprotéine majeure d'enveloppe d'un rétrovirus HIV-2,
- il n'est pas reconnu par des anticorps présents dans le sérum d'un patient infecté par un rétrovirus humain HIV-1, lesdits anticorps reconnaissant la glycoprotéine majeure d'enveloppe d'un rétrovirus HIV-1,
- il contient un nombre de résidus d'acides aminés n'excédant pas 40.

3. Peptide selon la revendication 1 ou 2 caractérisé par l'une des formules : 
XRV-AIEKYL-DQA-LN-WGCAFRQVCZ
XAIEKYL-DZ
 dans laquelle X et Z sont des groupements OH ou NH₂ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :

RVTAIEKYLQDQARLNSWGCAFRQVC
AIEKYLQDQ
RVSAIEKYLKDQAQLNAWGCAFRQVC
AIEKYLKDQ.

4. Peptide selon la revendication 1 ou 2 caractérisé par l'une des formules : 
X-LE-AQIQQEKNMYELQKLNSWZ
XQIQQEKNZ
 dans laquelle X et Z sont des groupements OH ou NH₂ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :

SLEQAQIQQEKNMYELQKLNSW
QIQQEKN
LLEEAQIQQEKNMYELQKLNSW.

5. Peptide selon la revendication 1 ou 2 caractérisé par l'une des formules : 
XELGDYKLVEITPIG-APT--KR-----Z
XYKLVEITPIG-APT--KRZ
 dans laquelle X et Z sont des groupements OH ou NH₂ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :

ELGDYKLVEITPIGFAPTKEKRYSSAH
YKLVEITPIGFAPTKEK
ELGDYKLVEITPIGLAPTNVKRYTTG-
YKLVEITPIGLAPTNVK.

6. Peptide selon la revendication 1 caractérisé par l'une des formules : 
X----VTV-YGVP-W--AT--LFCA-Z
XVTV-YGVP-W--ATZ
 dans laquelle X et Z sont des groupements OH ou NH₂ ou, dans la mesure ou les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :

CTQYVTVFYGVPTWKNATIPLFCAT
VTVFYGVPTWKNAT
CIQYVTVFYGVPAWRNATIPLFCAT
VTVFYGVPAWRNAT
EKLWVTVYYGVPVWKEATTTLFCAS
VTVYYGVPVWKEAT.

7. Peptide selon la revendication 6 caractérisé par

CTQYVTVFYGVPTWKNATIPLFCAT
VTVFYGVPTWKNAT
CIQYVTVFYGVPAWRNATIPLFCAT
VTVFYGVPAWRNAT
EKLWVTVYYGVPVWKEATTTLFCAS
VTVYYGVPVWKEAT
EDLWVTVYYGVPVWKEATTTLFCAS
VTVYYGVPVWKEAT
DNLWVTVYYGVPVWKEATTTLFCAS
VTVYYGVPVWKEAT.

8. Peptide selon la revendication 1 caractérisé par l'une des formules : 
X----E--L-NVTE-F--W-NZ
XL-NVTE-FZ
 dans laquelle X et Z sont des groupements OH ou NH₂ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :

DDYQEITL-NVTEAFDAWNN
L-NVTE
DDYSELAL-NVTESFDAWEN
PNPQEVVLVNVTENFNMWKN
LVNVTE.

9. Peptide selon la revendication 8 caractérisé par l'une des formules :

DDYQEITL-NVTEAFDAWNN
L-NVTEAF
DDYSELAL-NVTESFDAWEN
L-NVTESF
PNPQEVVLVNVTENFNMWKN
LVNVTENF
PNPQEIELENVTEGFNMWKN
LENVTEGF
PNPQEIALENVTENFNMWKN
LENVTENF.

10. Peptide selon la revendication 1 caractérisé par l'une des formules : 
XL---S-KPCVKL-PLC----Z
XKPCVKLTPLCVZ
XS-KPCVKLTPLCVZ
 dans laquelle X et Z sont des groupements OH ou NH₂ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :

LFETSIKPCVKLTPLCVAMK
LFETSIKPCVKLSPLCITMR
LWDQSLKPCVKLTPLCVSLK
KPCVKLTPLCV
KPCVKLSPLCI
SLKPCVKLTPLCV.

11. Peptide selon la revendication 10 caractérisé par l'une des structures suivantes :

LFETSIKPCVKLTPLCVAMK
LFETSIKPCVKLSPLCITMR
LWDQSLKPCVKLTPLCVSLK
LWDQSLKPCVKLTPLCVTLN
PCVKLTPLCV
KPCVKLSPLCI

12. Peptide selon la revendication 1 caractérise en ce qu'il contient la structure de base : 
X---N-S-I---C-Z
XN-S-I-Z
 dans laquelle X et Z sont des groupements OH ou NH₂ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :

NHCNTSVITESCD
NTSVIT
NHCNTSVIQECCD
NTSVIQ
TSCNTSVITQACP
NTSVIT.

13. Peptide selon la revendication 12 caractérisé par l'une des formules suivantes :

NHCNTSVITESCD
NTSVIT
NHCNTSVIQECCD
NTSVIQ
TSCNTSVITQACP
NTSVIT
INCNTSVITQACP
NTSVIT
INCNTSAITQACP
NTSAIT.

14. Peptide selon la revendication 1 caractérisé par l'une des formules suivantes :
XYC-P-G-A-L-C-N-TZ
dans laquelle X et Z sont des groupements OH ou NH₂ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :
YCAPPGYALLRC-NDT
YCAPAGFAILKCNNKT.

15. Peptide selon la revendication 14 caractérisé par l'une des formules :
YCAPPGYALLRC-NDT
YCAPAGFAILKCNNKT
YCAPAGFAILKCNDKK
YCAPAGFAILKCRDKK.

16. Peptide selon la revendication 1 caractérisé par la formule :
X------A-C------W--Z
dans laquelle X et Z sont des groupements OH ou NH₂ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :
NKRPRQAWCWFKG-KWKD
NERPKQAWCRFGG-NWKE
N--MRQAHCNISRAKWNA.

17. Peptide selon la revendication 16 caractérisé par la formule suivante :
NKRPRQAWCWFKG-KWKD
NERPKQAWCRFGG-KWKE
N--MRQAHCNISRAKWNA
D--IRRAYCTINETEWDK
I--IGQAHCNISRAQWSK.

18. Peptide selon la revendication 1 caractérisé par la formule suivante : 
X-G-DPE------NC-GEF-YC------NZ
XNC-GEF-YC-Z
 dans laquelle X et Z sont des groupements OH ou NH₂ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :

KGSDPEVAYMWTNCRGEFLYCNMTWFLN
NCRGEFLYCN
GG-DPEVTFMWTNCRGEFLYCKMNWFLN
NCRGEFLYCK
-GGDPEIVTHSFNCGGEFFYCNSTQLFN
NCGGEFFYCN.

19. Peptide selon la revendication 18 caractérisé par l'une des structures suivantes :

KGSDPEVAYMWTNCRGEFLYCNMTWFLN
NCRGEFLYCN
GG-DPEVTFMWTNCRGEFLYCKMNWFLN
NCRGEFLYCK
-GGDPEIVTHSFNCGGEFFYCNSTQLFN
NCGGEFFYCN
-GGDPEITTHSFNCRGEFFYCNTSKLFN
NCRGEFFYCN
-GGDPEITTHSFNCGGEFFYCNTSGLFN
NCGGEFFYCN.

20. Peptide selon la revendication 1 caractérisé par l'une des formules suivantes : 
X-----C-I-Q-I------G---YZ
      XC-I-Q-IZ
 dans laquelle X et Z sont des groupements OH ou NH₂ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :

RNYAPCHIKQIINTWHKVGRNVY
     CHIKQII
RNYVPCHIRQIINTWHKVGKNVY
     CHIRQII
TITLPCRIKQFINMWQEVGKAMY
     CRIKQFI.

21. Peptide selon la revendication 20 caractérisé par l'une des structures suivantes :

RNYAPCHIKQIINTWHKVGRNVY
CHIKQII
RNYVPCHIRQIINTWHKVGKNVY
CHIRQII
TITLPCRIKQFINMWQEVGKAMY

CRIKQFI
SITLPCRIKQIINMWQKTCKAMY
CRIKQII
NITLQCRIKQIIKMVAGR-KAIY
CRIKQII.

22. Peptide antigénique gag1, caractérisé par l'une des structures de base :
XDCKLVLKGLGMNPTLEEMLTAZ
XDCKLVLKGLGTNPTLEEMLTAZ
dans laquelle X et Z sont des groupements OH ou NH₂ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini les propriétés immunologiques de l'une des séquences suivantes :
DCKLVLKGLGMNPTLEEMLTA
DCKLVLKGLGTNPTLEEMLTA.

23. Peptide reconnu par des anticorps dirigés contre des protéines de HIV-2, caractérisé en ce qu'il comprend tout ou partie des séquences d'acides aminés choisies parmi les séquences suivantes :
POL_{ROD} ou POL_{mac} représenté à la figure 5
Q_{ROD} ou Q_{mac} représenté à la figure 6
R_{ROD} ou R_{mac} représenté à la figure 7
X_{ROD} ou X_{mac} représenté à la figure 8
F_{ROD} ou F_{mac} représenté à la figure 9
TAT_{ROD} ou TAT_{mac} représenté à la figure 10
ART_{ROD} ou ART_{mac} représenté à la figure 11.

24. Peptide reconnu par des anticorps reconnaissant la glycoprotéine d'enveloppe des virus de la classe HIV-2 caractérisé en ce qu'il comprend tout ou partie de la séquence d'acides aminés ENV de SIV-1 représentée à la figure 3.

25. Peptide reconnu par des anticorps reconnaissant la protéine du noyau des virus de la classe HIV-2, caractérisé en ce qu'il comprend tout ou partie de la séquence d'acides aminés GAG de SIV-1 représentée à la figure 4.

26. Utilisation de peptides selon l'une quelconque des revendications 1 à 25 pour la détection in vitro dans un échantillon biologique, d'une infection par un rétrovirus HIV-2, ou par un rétrovirus HIV-1.

27. Utilisation de peptides selon l'une quelconque des revendications 1 à 5 dans des compositions immunogènes capables d'induire la production d'anticorps contre un rétrovirus HIV-2 ou contre un rétrovirus HIV-1.

28. Utilisation selon la revendication 26 ou la revendication 27, caractérisée en ce que les peptides répondent à la définition de l'une quelconque des revendications 1 à 25.

29. Utilisation pour la détection in vitro dans un échantillon biologique, d'une infection par un rétrovirus HIV-2, de peptides ayant les propriétés suivantes :
- il sont reconnus par des anticorps présents dans le sérum d'un patient infecté par un rétrovirus humain HIV-2, lesdits anticorps reconnaissant la glycoprotéine majeure d'enveloppe d'un rétrovirus HIV-2,
- il ne sont pas reconnus par des anticorps présents dans le sérum d'un patient infecté par un rétrovirus humain HIV-1, lesdits anticorps reconnaissant la glycoprotéine majeure d'enveloppe d'un rétrovirus HIV-1,
- ils contiennent un nombre de résidus d'acides aminés n'excédant pas 40.

30. Utilisation selon la revendication 29, caractérisée en ce que les peptides répondent à la définition de l'une quelconque des revendications 3 à 5.

31. Composition antigénique contenant au moins un peptide gag selon la revendication 25 ou gag1 selon la revendication 22 ou au moins un oligomère de ce peptide, caractérisée en ce qu'elle a la capacité d'être reconnue par des fluides biologiques d'origine humaine, notamment des sérums contenant des anticorps anti-HIV-2 et dans une certaine mesure des anticorps anti-HIV-1.

32. Composition antigénique contenant un peptide env selon la revendication 24 ou au moins un peptide selon les revendications 2, 3, 4 ou 5 ou au moins un oligomère de ce peptide, caractérisée en ce qu'elle reconnait spécifiquement la présence d'anticorps contre HIV-2.

33. Composition immunogène contenant tout ou partie du peptide env selon la revendication 24 ou au moins un peptide selon les revendications 1, 6 à 21 ou au moins un oligomère de ce peptide ou ce peptide sous forme conjuguée avec une molécule porteuse, en association avec un véhicule pharmaceutique acceptable pour la production de vaccins, caractérisée en ce qu'elle induit la production d'anticorps contre les susdits peptides en quantité suffisante pour inhiber efficacement les protéines du rétrovirus HIV-2, voire même le rétrovirus HIV-2 entier.

34. Composition immunogène selon la revendication 33, caractérisée en ce qu'elle contient au moins un peptide ou au moins un oligomère de ce peptide ou ce peptide sous forme conjuguée avec une molécule porteuse, choisi parmi env4, env5, env6 et env10.

35. Procédé de diagnostic in vitro de l'infection par HIV-2 dans un liquide biologique comprenant :
- la mise en contact de ce liquide biologique avec au moins un peptide selon l'une des revendications 1 à 5 ou 22 ou un conjugué de ces peptides avec une molécule porteuse, ou des peptides env ou gag selon les revendications 24 ou 25,
- la détection de la présence éventuelle d'un complexe antigène-anticorps par des méthodes physiques ou chimiques, dans ledit liquide biologique.

36. Procédé de diagnostic in vitro de l'infection par HIV-2 dans un liquide biologique selon la revendication 35, caractérisé en ce que la détection du complexe antigène-anticorps éventuellement formé est réalisée grâce à des tests immunoenzymatiques (du type ELISA) immunofluorescents (du type IFA) radioimmunologiques (du type RIA) ou des tests de radioimmunoprécipitation (du type RIPA).

37. Kit pour le diagnostic in vitro de l'infection par HIV-2 dans un liquide biologique caractérisé en ce qu'il comprend :
- une composition peptidique contenant un peptide selon l'une quelconque des revendications 1 à 5 ou 22 ou un mélange de ces peptides, ou un conjugué de ces peptides avec une molécule porteuse, ou des peptides env ou gag selon la revendication 24 ou 25,
- un réactif pour la constitution du milieu propice à la réalisation d'une réaction immunologique,
- un ou plusieurs réactifs éventuellement marqué pour la détection du complexe antigène-anticorps formé par la réaction immunologique,
- un liquide biologique de référence dépourvu d'anticorps reconnus par la susdite composition peptidique.

## Claims

1. Peptide characterized by the following properties:
- it is recognized by antibodies present in the serum of a patient infected with a human retrovirus HIV-2, the said antibodies recognizing the major envelope glycoprotein of a retrovirus HIV-2,
- it contains a number of amino acid residues not exceeding 40.

2. Peptide according to Claim 1, characterized by the following properties:
- it is recognized by antibodies present in the serum of a patient infected with a human retrovirus HIV-2, the said antibodies recognizing the major envelope glycoprotein of a retrovirus HIV-2,
- it is not recognized by antibodies present in the serum of a patient infected with a human retrovirus HIV-1, the said antibodies recognizing the major envelope glycoprotein of a retrovirus HIV-1,
- it contains a number of amino acid residues not exceeding 40.

3. Peptide according to Claim 1 or 2, characterized by one of the formulae: 
XRV-AIEKYL-DQA-LN-WGCAFRQVCZ
XAIEKYL-DZ
 in which X and Z are OH or NH₂ groups or, insofar as the immunological properties of the peptide lacking these groups is not essentially modified therefrom, groups comprising 1 to 5 amino acid residues, and each of the dashes corresponds to an aminoacycl residue chosen from those which make it possible to preserve in the above-defined peptide the immunological properties of one of the following sequences:

RVTAIEKYLQDQARLNSWGCAFRQVC
   AIEKYLQDQ
RVSAIEKYLKDQAQLNAWGCAFRQVC
   AIEKYLKDQ.

4. Peptide according to Claim 1 or 2, characterized by one of the formulae: 
X-LE-AQIQQEKNMYELQKLNSWZ
XQIQQEKNZ
 in which X and Z are OH or NH₂ groups or, insofar as the immunological properties of the peptide lacking these groups is not essentially modified therefrom, groups comprising 1 to 5 amino acid residues, and each of the dashes corresponds to an aminoacycl residue chosen from those which make it possible to preserve in the above-defined peptide the immunological properties of one of the following sequences:

SLEQAQIQQEKNMYELQKLNSW
QIQQEKN
LLEEAQIQQEKNMYELQKLNSW.

5. Peptide according to Claim 1 or 2, characterized by one of the formulae: 
XELGDYKLVEITPIG-APT--KR-----Z
XYKLVEITPIG-APT--KRZ
 in which X and Z are OH or NH₂ groups or, insofar as the immunological properties of the peptide lacking these groups is not essentially modified therefrom, groups comprising 1 to 5 amino acid residues, and each of the dashes corresponds to an aminoacycl residue chosen from those which make it possible to preserve in the above-defined peptide the immunological properties of one of the following sequences:

ELGDYKLVEITPIGFAPTKEKRYSSAH
YKLVEITPIGFAPTKEK
ELGDYKLVEITPIGLAPTNVKRYTTG-
YKLVEITPIGLAPTNVK.

6. Peptide according to Claim 1, characterized by one of the formulae: 
X----VTV-YGVP-W--AT--LFCA-Z
XVTV-YGVP-W--ATZ
 in which X and Z are OH or NH₂ groups or, insofar as the immunological properties of the peptide lacking these groups is not essentially modified therefrom, groups comprising 1 to 5 amino acid residues, and each of the dashes corresponds to an aminoacycl residue chosen from those which make it possible to preserve in the above-defined peptide the immunological properties of one of the following sequences:

CTQYVTVFYGVPTWKNATIPLFCAT
VTVFYGVPTWKNAT
CIQYVTVFYGVPAWRNATIPLFCAT
VTVFYGVPAWRNAT
EKLWVTVYYGVPVWKEATTTLFCAS
VTVYYGVPVWKEAT.

7. Peptide according to Claim 6, characterized by:

CTQYVTVFYGVPTWKNATIPLFCAT
VTVFYGVPTWKNAT
CIQYVTVFYGVPAWRNATIPLFCAT
VTVFYGVPAWRNAT
EKLWVTVYYGVPVWKEATTTLFCAS
VTVYYGVPVWKEAT
EDLWVTVYYGVPVWKEATTTLFCAS
VTVYYGVPVWKEAT
DNLWVTVYYGVPVWKEATTTLFCAS
VTVYYGVPVWKEAT.

8. Peptide according to Claim 1, characterized by one of the formulae: 
X----E--L-NVTE-F--W-NZ
XL-NVTE-FZ
 in which X and Z are OH or NH₂ groups or, insofar as the immunological properties of the peptide lacking these groups is not essentially modified therefrom, groups comprising 1 to 5 amino acid residues, and each of the dashes corresponds to an aminoacycl residue chosen from those which make it possible to preserve in the above-defined peptide the immunological properties of one of the following sequences:

DDYQEITL-NVTEAFDAWNN
L-NVTE
DDYSELAL-NVTESFDAWEN
PNPQEVVLVNVTENFNMWKN
LVNVTE.

9. Peptide according to Claim 8, characterized by one of the formulae:

DDYQEITL-NVTEAFDAWNN
L-NVTEAF
DDYSELAL-NVTESFDAWEN
L-NVTESF
PNPQEVVLVNVTENFNMWKN
LVNVTENF
PNPQEIELENVTEGFNMWKN
LENVTEGF
PNPQEIALENVTENFNMWKN
LENVTENF.

10. Peptide according to Claim 1, characterized by one of the formulae: 
XL---S-KPCVKL-PLC----Z
XKPCVKLTPLCVZ
XS-KPCVKLTPLCVZ
 in which X and Z are OH or NH₂ groups or, insofar as the immunological properties of the peptide lacking these groups is not essentially modified therefrom, groups comprising 1 to 5 amino acid residues, and each of the dashes corresponds to an aminoacycl residue chosen from those which make it possible to preserve in the above-defined peptide the immunological properties of one of the following sequences:

LFETSIKPCVKLTPLCVAMK
LFETSIKPCVKLSPLCITMR
LWDQSLKPCVKLTPLCVSLK
KPCVKLTPLCV
KPCVKLSPLCI
SLKPCVKLTPLCV.

11. Peptide according to Claim 10, characterized by one of the following structures:

LFETSIKPCVKLTPLCVAMK
LFETSIKPCVKLSPLCITMR
LWDQSLKPCVKLTPLCVSLK
LWDQSLKPCVKLTPLCVTLN
PCVKLTPLCV
KPCVKLSPLCI.

12. Peptide according to Claim 1, characterized in that it contains the basic structure: 
X---N-S-I---C-Z
XN-S-I-Z
 in which X and Z are OH or NH₂ groups or, insofar as the immunological properties of the peptide lacking these groups is not essentially modified therefrom, groups comprising 1 to 5 amino acid residues, and each of the dashes corresponds to an aminoacycl residue chosen from those which make it possible to preserve in the above-defined peptide the immunological properties of one of the following sequences:

NHCNTSVITESCD
NTSVIT
NHCNTSVIQECCD
NTSVIQ
TSCNTSVITQACP
NTSVIT.

13. Peptide according to Claim 12, characterized by one of the following formulae:

NHCNTSVITESCD
NTSVIT
NHCNTSVIQECCD
NTSVIQ
TSCNTSVITQACP
NTSVIT
INCNTSVITQACP
NTSVIT
INCNTSAITQACP
NTSAIT.

14. Peptide according to Claim 1, characterized by one of the following formulae:
XYC-P-G-A-L-C-N-TZ
in which X and Z are OH or NH₂ groups or, insofar as the immunological properties of the peptide lacking these groups is not essentially modified therefrom, groups comprising 1 to 5 amino acid residues, and each of the dashes corresponds to an aminoacycl residue chosen from those which make it possible to preserve in the above-defined peptide the immunological properties of one of the following sequences:
YCAPPGYALLRC-NDT
YCAPAGFAILKCNNKT.

15. Peptide according to Claim 14, characterized by one of the formulae:
YCAPPGYALLRC-NDT
YCAPAGFAILKCNNKT
YCAPAGFAILKCNDKK
YCAPAGFAILKCRDKK.

16. Peptide according to Claim 1, characterized by the formula:
X------A-C------W--Z
in which X and Z are OH or NH₂ groups or, insofar as the immunological properties of the peptide lacking these groups is not essentially modified therefrom, groups comprising 1 to 5 amino acid residues, and each of the dashes corresponds to an aminoacycl residue chosen from those which make it possible to preserve in the above-defined peptide the immunological properties of one of the following sequences:
NKRPRQAWCWFRG-KWKD
NERPKQAWCRFGG-NWKE
N-MRQAHCNISRAKWNA.

17. Peptide according to Claim 16, characterized by the following formula:
NKRPRQAWCWFKG-KWKD
NERPKQAWCRFGG-KWFE
N--MRQAHCNISRAKWNA
D--IRRAYCTINETEWDK
I--IGQAHCNISRAQWSK.

18. Process according to Claim 1, characterized by the following formula: 
X-G-DPE------NC-GEF-YC------NZ
XNC-GEF-YC-Z
 in which X and Z are OH or NH₂ groups or, insofar as the immunological properties of the peptide lacking these groups is not essentially modified therefrom, groups comprising 1 to 5 amino acid residues, and each of the dashes corresponds to an aminoacycl residue chosen from those which make it possible to preserve in the above-defined peptide the immunological properties of one of the following sequences:

KGSDPEVAYMWTNCRGEFLYCNMTWFLN
NCRGEFLYCN
GG-DPEVTFMWTNCRGEFLYCKMNWFLN
NCRGEFLYCK
-GGDPEIVTHSFNCGGEFFYCNSTQLFN
NCGGEFFYCN.

19. Peptide according to Claim 18, characterized by one of the following structures:

KGSDPEVAYMWTNCRGEFLYCNMTWFLN
NCRGEFLYCN
GG-DPEVTFMWTNCRGEFLYCKMNWFLN
NCRGEFLYCK
-GGDPEIVTHSFNCGGEFFYCNSTQLFN
NCGGEFFYCN
-GGDPEITTHSFNCRGEFFYCNTSKLFN
NCRGEFFYCN
-GGDPEITTHSFNCGGEFFYCNTSGLFN
NCGGEFFYCN.

20. Peptide according to Claim 1, characterized by one of the following formulae: 
X-----C-I-Q-I------G---YZ
      XC-I-Q-IZ
 in which X and Z are OH or NH₂ groups or, insofar as the immunological properties of the peptide lacking these groups is not essentially modified therefrom, groups comprising 1 to 5 amino acid residues, and each of the dashes corresponds to an aminoacycl residue chosen from those which make it possible to preserve in the above-defined peptide the immunological properties of one of the following sequences:

RNYAPCHIKQIINTWHKVGRNVY
CHIKQII
RNYVPCHIRQIINTWHKVGKNVY
CHIRQII
TITLPCRIKQFINMWQEVGKAMY
CRIKQFI.

21. Peptide according to Claim 20, characterized by one of the following structures:

RNYAPCHIKQIINTWHKVGRNVY
CHIKQII
RNYVPCHIRQIINTWHKVGKNVY
CHIRQII
TITLPCRIKQFINMWQEVGKAMY
CRIKQFI
SITLPCRIKQIINMWQKTCKAMY
CRIKQII
NITLQCRIKQIIKMVAGR-KAIY
CRIKQII.

22. Antigenic peptide gag1, characterized by one of the basic structures:
XDCKLVLKGLGMNPTLEEMLTAZ
XDCKLVLKGLGTNPTLEEMLTAZ
in which X and Z are OH or NH₂ groups or, insofar as the immunological properties of the peptide lacking these groups is not essentially modified therefrom, groups comprising 1 to 5 amino acid residues, and in which each of the dashes corresponds to an aminoacycl residue chosen from those which make it possible to preserve in the above-defined peptide the immunological properties of either of the following sequences:
DCKLVLKGLGMNPTLEEMLTA
DCKLVLKGLGTNPTLEEMLTA.

23. Peptide recognized by antibodies directed against HIV-2 proteins, characterized in that it comprises all or part of the amino acid sequences chosen from the following sequences:
POL_{ROD} or POL_{mac} represented in Figure 5
Q_{ROD} or Q_{mac} represented in Figure 6
R_{ROD} or R_{mac} represented in Figure 7
X_{ROD} or X_{mac} represented in Figure 8
F_{ROD} or F_{mac} represented in Figure 9
TAT_{ROD} or TAT_{mac} represented in Figure 10
ART_{ROD} or ART_{mac} represented in Figure 11.

24. Peptide recognized by antibodies recognizing the envelope glycoprotein of viruses of the HIV-2 class, characterized in that it comprises all or part of the ENV amino acid sequence of SIV-1 represented in Figure 3.

25. Peptide recognized by antibodies recognizing the core protein of viruses of the HIV-2 class, characterized in that it comprises all or part of the GAG amino acid sequence of SIV-1 represented in Figure 4.

26. Use of peptides according to any one of Claims 1 to 25, for the in vitro detection, in a biological sample, of an infection by a HIV-2 retrovirus, or by a HIV-1 retrovirus.

27. Use of peptides according to any one of Claims 1 to 5, in immunogenic compositions capable of inducing the production of antibodies against a HIV-2 retrovirus or against a HIV-1 retrovirus.

28. Use according to Claim 26 or Claim 27, characterized in that the peptides correspond to the definition of any one of Claims 1 to 25.

29. Use for the in vitro detection in a biological sample, of an infection by a HIV-2 retrovirus, of peptides having the following properties:
- they are recognized by antibodies present in the serum of a patient infected with a human retrovirus HIV-2, the said antibodies recognizing the major envelope glycoprotein of a HIV-2 retrovirus,
- they are not recognized by antibodies present in the serum of a patient infected with a human retrovirus HIV-1, the said antibodies recognizing the major envelope glycoprotein of a HIV-1 retrovirus,
- they contain a number of amino acid residues not exceeding 40.

30. Use according to Claim 29, characterized in that the peptides correspond to the definition of any one of Claims 3 to 5.

31. Antigenic composition containing at least one gag peptide according to Claim 25 or one gag1 peptide according to Claim 22 or at least one oligomer of this peptide, characterized in that it has the capacity to be recognized by biological fluids of human origin, especially sera containing anti-HIV-2 antibodies and to a certain extent anti-HIV-1 antibodies.

32. Antigenic composition containing the env peptide according to Claim 24 or at least one peptide according to Claims 2, 3, 4 or 5 or at least one oligomer of this peptide, characterized in that it specifically recognizes the presence of antibodies against HIV-2.

33. Immunogenic composition containing all or part of the env peptide according to Claim 24 or at least one peptide, according to Claims 1, 6 to 21 or at least one oligomer of this peptide or this peptide in the form of a conjugate with one carrier molecule, in association with a pharmaceutical vehicle acceptable for the production of vaccines, characterized in that it induces the production of antibodies against the abovementioned peptides in a quantity sufficient to effectively inhibit the HIV-2 retrovirus proteins or even the whole HIV-2 retrovirus.

34. Immunogenic composition according to Claim 33, characterized in that it contains at least one peptide or at least one oligomer of this peptide or this peptide in the form of a conjugate with a carrier molecule chosen from env4, env5, env6 and env10.

35. Process for the in vitro diagnosis of HIV-2 infection in a biological fluid comprising:
- the placing of this biological fluid in contact with at least one peptide according to one of Claims 1 to 5 or 22 or a conjugate of these peptides with a carrier molecule or env or gag peptides according to Claim 24 or 25,
- the detection of the possible presence of an antigen-antibody complex by physical or chemical methods, in the said biological fluid.

36. Process for the in vitro diagnosis of HIV-2 infection in a biological fluid according to Claim 35, characterized in that the detection of the antigen-antibody complex which may be formed is performed by means of immunoenzymatic tests (of the ELISA type), immunofluorescent tests (of the IFA type), radioimmunological tests (of the RIA type) or radioimmunoprecipitation tests (of the RIPA type).

37. Kit for the in vitro diagnosis of HIV-2 infection in a biological fluid characterized in that it comprises:
- a peptide composition containing a peptide according to one of Claims 1 to 5, 22, or a mixture of these peptides, or a conjugate of these peptides with a carrier molecule, or the env or gag peptides according to Claim 24 or 25,
- a reagent for preparing the medium suitable for carrying out an immunological reaction,
- one or more optionally labelled reagents for the detection of the antigen-antibody complex produced by the immunological reaction,
- a reference biological fluid free of antibodies recognized by the abovementioned peptide composition.

## Patentansprüche

1. Peptid, gekennzeichnet durch folgende Eigenschaften :
- es wird von Antikörpern erkannt, die im Serum eines Patienten vorhanden sind, der durch ein humanes Retrovirus HIV-2 infiziert ist; die besagten Antikörper erkennen das größere Glykoprotein in der Hülle eines Retrovirus HIV-2,
- es enthält höchstens 40 Aminosäurereste.

2. Peptid gemäß Anspruch 1, gekennzeichnet durch folgende Eigenschaften :
- es wird von Antikörpern erkannt, die im Serum eines Patienten vorhanden sind, der durch ein humanes Retrovirus HIV-2 infiziert ist; die besagten Antikörper erkennen das größere Glykoprotein in der Hülle eines Retrovirus HIV-2,
- es wird nicht erkannt von Antikörpern, die im Serum eines Patienten vorhanden sind, der durch ein humanes Retrovirus HIV-1 infiziert ist; die besagten Antikörper erkennen das größere Glykoprotein in der Hülle eines Retrovirus HIV-1,
- es enthält höchstens 40 Aminosäurereste.

3. Peptid gemäß Anspruch 1 oder 2, gekennzeichnet durch eine der Formeln: 
XRV-AIEKYL-DQA-LN-WGCAFRQVCZ
XAIEKYL-DZ
 in der X und Z OH- oder NH₂-Gruppen sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immunologischen Eigenschaften einer der folgenden Sequenzen zu behalten :

RVTAIEKYLQDQARLNSWGCAFRQVC
AIEKYLQDQ
RVSAIEKYLKDQAQLNAWGCAFRQVC
AIEKYLKDQ

4. Peptid gemäß Anspruch 1 oder 2, gekennzeichnet durch eine der Formeln: 
X-LE-AQIQQEKNMYELQKLNSWZ
XQIQQEKNZ
 in der X und Z OH- oder NH₂-Gruppen sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immunologischen Eigenschaften einer der folgenden Sequenzen zu behalten :

SLEQAQIQQEKNMYELQKLNSW
QIQQEKN
LLEEAQIQQEKNMYELQKLNSW.

5. Peptid gemäß Anspruch 1 oder 2, gekennzeichnet durch eine der Formeln : 
XELGDYKLVEITPIG-APT--KR-----Z
XYKLVEITPIG-APT--KRZ
 in der X und Z OH- oder NH₂-Gruppen sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immunologischen Eigenschaften einer der folgenden Sequenzen zu behalten :

ELGDYKLVEITPIGFAPTKEKRYSSAH
YKLVEITPIGFAPTKEK
ELGDYKLVEITPIGLAPTNVKRYTTG-
YKLVEITPIGLAPTNVK.

6. Peptid gemäß Anspruch 1, gekennzeichnet durch eine der Formeln : 
X----VTV-YGVP-W--AT--LFCA-Z
XVTV-YGVP-W--ATZ
 in der X und Z OH- oder NH₂-Gruppen sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immunologischen Eigenschaften einer der folgenden Sequenzen zu behalten :

CTQYVTVFYGVPTWKNATIPLFCAT
VTVFYGVPTWKNAT
CIQYVTVFYGVPAWRNATIPLFCAT
VTVFYGVPAWRNAT
EKLWVTVYYGVPVWKEATTTLFCAS
VTVYYGVPVWKEAT.

7. Peptid gemäß Anspruch 6, gekennzeichnet durch

CTQYVTVFYGVPTWKNATIPLFCAT
VTVFYGVPTWKNAT
CIQYVTVFYGVPAWRNATIPLFCAT
VTVFYGVPAWRNAT
EKLWVTVYYGVPVWKEATTTLFCAS
VTVYYGVPVWKEAT
EDLWVTVYYGVPVWKEATTTLFCAS
VTVYYGVPVWKEAT
DNLWVTVYYGVPVWKEATTTLFCAS
VTVYYGVPVWKEAT.

8. Peptid gemäß Anspruch 1, gekennzeichnet durch eine der Formeln : 
X----E--L-NVTE-F--W-NZ
XL-NVTE-FZ
 in der X und Z OH- oder NH₂-Gruppen sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immunologischen Eigenschaften einer der folgenden Sequenzen zu behalten :

DDYQEITL-NVTEAFDAWNN
L-NVTE
DDYSELAL-NVTESFDAWEN
PNPQEVVLVNVTENFNMWKN
LVNVTE.

9. Peptid gemäß Anspruch 8, gekennzeichnet durch eine der Formeln :

DDYQEITL-NVTEAFDAWNN
L-NVTEAF
DDYSELAL-NVTESFDAWEN
L-NVTESF
PNPQEVVLVNVTENFNMWKN
LVNVTENF
PNPQEIELENVTEGFNMWKN
LENVTEGF
PNPQEIALENVTENFNMWKN
LENVTENF.

10. Peptid gemäß Anspruch 1, gekennzeichnet durch eine der Formeln : 
XL---S-KPCVKL-PLC----Z
XKPCVKLTPLCVZ
XS-KPCVKLTPLCVZ
 in der X und Z OH- oder NH₂-Gruppen sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immunologischen Eigenschaften einer der folgenden Sequenzen zu behalten :

LFETSIKPCVKLTPLCVAMK
LFETSIKPCVKLSPLCITMR
LWDQSLKPCVKLTPLCVSLK
KPCVKLTPLCV
KPCVKLSPLCI
SLKPCVKLTPLCV.

11. Peptid gemäß Anspruch 10, gekennzeichnet durch eine der folgenden Strukturen :

LFETSIKPCVKLTPLCVAMK
LFETSIKPCVKLSPLCITMR
LWDQSLKPCVKLTPLCVSLK
LWDQSLKPCVKLTPLCVTLN
PCVKLTPLCV
KPCVKLSPLCI.

12. Peptid gemäß Anspruch 1, gekennzeichnet dadurch, daß es die folgende Grundstruktur enthält : 
X---N-S-I---C-Z
XN-S-I-Z
 in der X und Z OH- oder NH₂-Gruppen sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immunologischen Eigenschaften einer der folgenden Sequenzen zu behalten :

NHCNTSVITESCD
NTSVIT
NHCNTSVIQECCD
NTSVIQ
TSCNTSVITQACP
NTSVIT.

13. Peptid gemäß Anspruch 12, gekennzeichnet durch eine der folgenden Formeln :

NHCNTSVITESCD
NTSVIT
NHCNTSVIQECCD
NTSVIQ
TSCNTSVITQACP
NTSVIT
INCNTSVITQACP
NTSVIT

INCNTSAITQACP
NTSAIT.

14. Peptid gemäß Anspruch 1, gekennzeichnet durch eine der folgenden Formeln :
XYC-P-G-A-L-C-N-TZ
in der X und Z OH- oder NH₂-Gruppen sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immunologischen Eigenschaften einer der folgenden Sequenzen zu behalten :
YCAPPGYALLRC-NDT
YCAPAGFAILKCNNKT.

15. Peptid gemäß Anspruch 14, gekennzeichnet durch eine der Formeln :
YCAPPGYALLRC-NDT
YCAPAGFAILKCNNKT
YCAPAGFAILKCNDKK
YCAPAGFAILKCRDKK.

16. Peptid gemäß Anspruch 1, gekennzeichnet durch die Formel:
X------A-C------W--Z
in der X und Z OH- oder NH₂-Gruppen sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immonologischen Eigenschaften einer der folgenden Sequenzen zu behalten :
NKRPRQAWCWFKG-KWKD
NERPKQAWCRFGG-NWKE
N--MRQAHCNISRAKWNA.

17. Peptid gemäß Anspruch 16, gekennzeichnet durch die folgende Formel :
NKRPRQAWCWFKG-KWKD
NERPKQAWCRFGG-KWKE
N--MRQAHCNISRAKWNA
D--IRRAYCTINETEWDK
I--IGQAHCNISRAQWSK.

18. Peptid gemäß Anspruch 1, gekennzeichnet durch die folgende Formel : 
X-G-DPE------NC-GEF-YC------NZ
XNC-GEF-YC-Z
 in der X und Z OH- oder NH₂-Gruppenm sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immunologischen Eigenschaften einer der folgenden Sequenzen zu behalten :

KGSDPEVAYMWTNCRGEFLYCNMTWFLN
NCRGEFLYCN
GG-DPEVTFMWTNCRGEFLYCKMNWFLN
NCRGEFLYCK
-GGDPEIVTHSFNCGGEFFYCNSTQLFN
NCGGEFFYCN.

19. Peptid gemäß Anspruch 18, gekennzeichnet durch eine der folgenden Strukturen :

KGSDPEVAYMWTNCRGEFLYCNMTWFLN
NCRGEFLYCN
GG-DPEVTFMWTNCRGEFLYCKMNWFLN
NCRGEFLYCK
-GGDPEIVTHSFNCGGEFFYCNSTQLFN
NCGGEFFYCN
-GGDPEITTHSFNCRGEFFYCNTSKLFN
NCRGEFFYCN
-GGDPEITTHSFNCGGEFFYCNTSGLFN
NCGGEFFYCN.

20. Peptid gemäß Anspruch 1, gekennzeichnet durch eine der folgenden Formeln : 
X-----C-I-Q-I------G---YZ
XC-I-Q-IZ
 in der X und Z OH- oder NH₂-Gruppen sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immunologischen Eigenschaften einer der folgenden Sequenzen zu behalten :

RNYAPCHIKQIINTWHKVGRNVY
CHIKQII
RNYVPCHIRQIINTWHKVGKNVY
CHIRQII
TITLPCRIKQFINMWQEVGKAMY
CRIKQFI.

21. Peptid gemäß Anspruch 20, gekennzeichnet durch eine der folgenden Strukturen :

RNYAPCHIKQIINTWHKVGRNVY
CHIKQII
RNYVPCHIRQIINTWHKVGKNVY
CHIRQII
TITLPCRIKQFINMWQEVGKAMY
CRIKQFI
SITLPCRIKQIINMWQKTCKAMY
CRIKQII
NITLQCRIKQIIKMVAGR-KAIY
CRIKQII.

22. Antigenpeptid gag1, gekennzeichnet durch eine der Grundstrukturen :
XDCKLVLKGLGMNPTLEEMLTAZ
XDCKLVLKGLGTNPTLEEMLTAZ
in dem X und Z OH- und NH₂-Gruppen sind oder, in dem Maße wie die immunologischen Eigenschaften des Peptids ohne diese Gruppen sich nicht wesentlich ändern, Gruppen, die 1 bis 5 Aminosäurereste enthalten, und jeder Bindestrich einem Aminoacylrest entspricht, der unter denen ausgewählt wurde, die es dem oben definierten Peptid ermöglichen, die immunologischen Eigenschaften einer der folgenden Sequenzen zu behalten :
DCKLVLKGLGMNPTLEEMLTA
DCKLVLKGLGTNPTLEEMLTA.

23. Von den gegen Proteine des HIV-2 gerichteten Antikörpern erkanntes Peptid, dadurch gekennzeichnet, daß es alle oder einen Teil der Aminosäuresequenzen enthält, die unter folgenden Sequenzen ausgewählt wurden :
POL_{ROD} oder POL_{mac}, dargestellt in Figur 5
Q_{ROD} oder Q_{mac}, dargestellt in Figur 6
R_{ROD} oder R_{mac}, dargestellt in Figur 7
X_{ROD} oder X_{mac}, dargestellt in Figur 8
F_{ROD} oder F_{mac}, dargestellt in Figur 9
TAT_{ROD} oder TAT_{mac}, dargestellt in Figur 10
ART_{ROD} oder ART_{mac}, dargestellt in Figur 11.

24. Peptid, das von Antikörpern erkannt wird, die das Glykoprotein in der Hülle von Viren der Klasse HIV-2 erkennen, dadurch gekennzeichnet, daß es die ganze oder einen Teil der Aminosäuresequenz ENV von SIV-1 enthält, die in Figur 3 wiedergegeben wird.

25. Peptid, das von Antikörpern erkannt wird, die das Protein des Kerns der Viren der Klasse HIV-2 erkennen, dadurch gekennzeichnet, daß es die ganze oder einen Teil der Aminosäuresequenz GAG von SIV-1 enthält, die in Figur 4 wiedergegeben wird.

26. Verwendung von Peptiden gemäß irgendeinem der Ansprüche 1 bis 25 für die Erkennung in vitro einer durch ein Retrovirus HIV-2 oder HIV-1 hervorgerufenen Infektion in einer biologischen Probe.

27. Verwendung von Peptiden gemäß irgendeinem der Ansprüche 1 bis 5 in immunogenen Zusammensetzungen, die in der Lage sind, die Produktion von gegen ein Retrovirus HIV-2 oder HIV-1 gerichteten Antikörpern zu induzieren.

28. Verwendung gemäß Anspruch 26 oder Anspruch 27, dadurch gekennzeichnet, daß die Peptide der Definition irgendeines der Ansprüche 1 bis 25 entsprechen.

29. Verwendung von Peptiden mit nachfolgenden Eigenschaften für die Erkennung in vitro einer durch ein Retrovirus HIV-2 hervorgerufenen Infektion in einer biologischen Probe :
- sie werden von Antikörpern erkannt, die im Serum eines Patienten vorhanden sind, der durch ein humanes Retrovirus HIV-2 infiziert wurde; die besagten Antikörper erkennen das größere Glykoprotein in der Hülle eines Retrovirus HIV-2,
- sie werden nicht erkannt von Antikörpern, die im Serum eines Patienten vorhanden sind, der durch ein humanes Retrovirus HIV-1 infiziert wurde; die besagten Antikörper erkennen das Glyko-protein in der Hülle eines Retrovirus HIV-1,
- sie enthalten höchstens 40 Aminosäurereste.

30. Verwendung gemäß Anspruch 29, dadurch gekennzeichnet, daß die Peptide der Definition irgendeines der Ansprüche 3 bis 5 entsprechen.

31. Antigene Zusammensetzung, die mindestens ein Peptid gag gemäß Anspruch 25 oder gag1 gemäß Anspruch 22 oder mindestens ein Oligomer dieses Peptids enthält, dadurch gekennzeichnet, daß es die Fähigkeit besitzt, von biologischen Flüssigkeiten menschlicher Herkunft erkannt zu werden, insbesondere von Seren, welche gegen HIV-2 gerichtete Antikörper und in einem gewissen Umfang gegen HIV-1 gerichtete Antikörper enthalten.

32. Antigene Zusammensetzung, die ein Peptid env gemäß Anspruch 24 oder zumindest ein Peptid gemäß den Ansprüchen 2, 3, 4 oder 5 oder mindestens ein Oligomer dieses Peptids enthält, dadurch gekennzeichnet, daß sie spezifisch das Vorhandensein gegen HIV-2 gerichteter Antikörper erkennt.

33. Immunogene Zusammensetzung, die das ganze oder einen Teil des Peptids env gemäß Anspruch 24 oder zumindest ein Peptid gemäß den Ansprüchen 1, 6 bis 21 oder zumindest ein Oligomer dieses Peptids oder dieses Peptid in konjugierter Form mit einem Trägermolekül in Verbindung mit einem für die Produktion von Impfstoffen akzeptablen pharmazeutischen Vehikel enthält, dadurch gekennzeichnet, daß sie die Produktion von gegen die oben genannten Peptide gerichteten Antikörpern in ausreichender Menge induziert, um die Proteine des Retrovirus HIV-2, ja sogar das ganze Retrovirus HIV-2 wirksam zu hemmen.

34. Immunogene Zusammensetzung gemaß Anspruch 33, dadurch gekennzeichnet, daß sie mindestens ein Peptid oder mindestens ein Oligomer dieses Peptids oder dieses Peptid in konjugierter Form mit einem Trägermolekül enthält, das unter env4, env5, env6 und env10 ausgewählt wurde.

35. Verfahren für die in vitro durchgeführte Diagnose der durch HIV-2 verursachten Infektion in einer biologischen Flüssigkeit, das beinhaltet :
- Herstellung des Kontakts dieser biologischen Flüssigkeit mit mindestens einem Peptid gemäß einem der Ansprüche 1 bis 5 oder 22 oder einem Konjugat dieser Peptide mit einem Trägermolekül, oder Peptiden env oder gag gemäß den Ansprüchen 24 oder 25,
- Erkennen des eventuellen Vorhandenseins eines Komplexes Antigen-Antikörper durch physikalische oder chemische Methoden in besagter biologischer Flüssigkeit.

36. Verfahren für die in vitro durchgeführte Diagnose der durch HIV-2 verursachten Infektion in einer biologischen Flüssigkeit gemäß Anspruch 35, dadurch gekennzeichnet, daß die Erkennung des eventuell gebildeten Komplexes Antigen-Antikörper realisiert wird dank immunoenzymatischer Tests (vom Typ ELISA), Immunofluoreszenztests (vom Typ IFA), radioimmunologischer Tests (vom Typ RIA) oder Radioimmunopräzipitationstests (vom Typ RIPA).

37. Kit für die in vitro durchgeführte Diagnose der durch HIV-2 verursachten Infektion in einer biologischen Flüssigkeit, dadurch gekennzeichnet, daß es besteht aus :
- einer Peptidkomposition, die ein Peptid gemäß irgendeinem der Ansprüche 1 bis 5 oder 22, oder eine Mischung dieser Peptide, oder ein Konjugat dieser Peptide mit einem Trägermolekül, oder Peptide env oder gag gemäß Anspruch 24 oder 25 enthält,
- einem Reagenz für die Bildung des für die Durchführung einer immunologischen Reaktion günstigen Milieus,
- ein oder mehrere, eventuell markierte Reagenzien für die Erkennung des durch die immunologische Reaktion gebildeten Komplexes Antigen-Antikörper,
- eine biologische Vergleichslösung, die frei ist von durch die oben genannte Peptidkomposition erkannten Antikörpern.
